(19) Europäisches Patentamt
European Patent Office
Office européen des brevets

(11) Veröffentlichungsnummer: **0 305 320 B1**

(12) # EUROPÄISCHE PATENTSCHRIFT

(45) Veröffentlichungstag der Patentschrift: **23.09.92**

(21) Anmeldenummer: **88730187.7**

(22) Anmeldetag: **23.08.88**

(51) Int. Cl.5: **C07C 229/28**, C07D 257/02, C07D 207/452, C07F 5/00, A61K 49/00, A61K 31/555

(54) **Mehrkernige substituierte Komplexbildner, Komplexe und Komplexsalze, Verfahren zu deren Herstellung und diese enthaltende pharmazeutische Mittel.**

(30) Priorität: **24.08.87 DE 3728525**

(43) Veröffentlichungstag der Anmeldung: **01.03.89 Patentblatt 89/09**

(45) Bekanntmachung des Hinweises auf die Patenterteilung: **23.09.92 Patentblatt 92/39**

(84) Benannte Vertragsstaaten: **AT BE CH DE ES FR GB GR IT LI LU NL SE**

(56) Entgegenhaltungen:
**EP-A- 0 186 947**
**WO-A-86/02352**
**WO-A-86/06384**
**DE-A- 3 401 052**

**CHEMICAL ABSTRACTS, Band 76, 1. Mai 1972, Seite 351, Nr. 104531j, Columbus, Ohio, US; I.V. PODGORNAYA et al.: "Complexing properties of a polycomplexon", & TR. INST. KHIM. AKAD. NAUK SSSR, URAL. FILIAL 1971, Nr. 21, 51-5**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Deutsch, Julius, Dr.**
**Horstweg 25**
**W-1000 Berlin 19(DE)**
Erfinder: **Conrad, Jürgen, Dr.**
**Sonnenallee 70**
**W-1000 Berlin 44(DE)**

EP 0 305 320 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft den in den Patentansprüchen gekennzeichneten Gegenstand, das heißt neue Komplexbildner, Komplexe und Komplexsalze, diese Verbindungen enthaltende Mittel, ihre Verwendung in Diagnostik und Therapie sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und der en Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotrope wie $^{99m}Tc$ für die Szintigraphie sind bekannt.

In der Patentschrift DE-OS 3401052 sind neuerdings paramagnetische Komplexsalz als Diagnostika, vorwiegend als NMR-Diagnostika vorgeschlagen worden.

Alle bisher bekannten Komplexe und deren Salze bereiten bei ihrer klinischen Anwendung Probleme im Hinblick auf die Verträglichkeit und/oder Selektivität der Bindung und/oder Stabilität. Diese Probleme sind umso ausgeprägter, je höher die aus den Komplexbildnern abgeleiteten Produkte dosiert werden müssen. Die an und für sich nützliche Anwendung schwerer Elemente als Bestandteile von parenteral zu verabreichenden Röntgenkontrastmitteln scheiterte bisher an der ungenügenden Verträglichkeit derartiger Verbindungen. Bei den bisher für die Kernspintomographie vorgeschlagenen oder geprüften paramagnetischen Substanzen ist der Abstand zwischen der wirksamen und der im Tierexperiment toxischen Do sis relativ eng, und/oder sie weisen eine geringe Organspezifizität und/oder Stabilität und/oder kontrastverstärkende Wirkung auf und/oder ihre Verträglichkeit ist unzureichend.

Der Ansatz, zumindest einen Teil dieser Probleme durch Verwendung von Komplexbildnern, die einerseits durch ionische Bindung an das jeweils geeignete Metall (siehe unten) sowie andererseits durch Bindung an eine funktionelle Gruppe oder ein als Carrier-Molekül dienendes nicht-toxisches und möglichst organ-spezifisches Makromolekül gebunden sind, zu lösen, was bisher nur sehr begrenzt erfolgreich.

Werden die funktionellen Gruppen des Komplexbildners zur Bindung des Moleküls an ein Makromolekül benutzt, so kommt es zu einer Abschwächung der Komplexstabilität, das heißt ein physiologisch nicht tolerierbarer Anteil der Metallionen des Makromolekül-Metallionen-Komplexes wird freigesetzt {C.H. Paik et al., J.Radioanal. Chem. 57,553 (1980), D.J. Hnatowich et al., J. Nucl. Med. 26,503 (1985)}.

Verwendet man andererseits als Edukte bifunktionelle Komplexbildner, das heißt Komplexbildner, die sowohl funktionelle Gruppen zur koordinativen Bindung des gewünschten Metallions als auch eine (andere) funktionelle Gruppe zur Bindung des Makromoleküls tragen, so treten nach dem jetzigen Stand der Technik ( C.F. Meases et al, Radioimmunoimaging and Radioimmunotherapie 1983, 185, Canadisches Patent No. 1 178 951) die verschiedensten gravierenden Nachteile auf; zum Beispiel geringe Stabilität der Komplexe, vielstufige schwierige Synthese der Komplexe, geringe Variationsmöglichkeiten der für die Bindung an das Makromolekül benötigten funktionellen Gruppe, Gefahr der Kontaminierung der Komplexbildner während ihrer Synthese mit Fremdmetallen, auf Grund zu geringer Lipophilie nur begrenzte Reaktionsmöglichkeiten der Komplexbildner, mit Ausbeuteminderung und zusätzlichen Reinigungsschritten verbundene notwendige intermediäre Blockade der funktionellen Gruppen der Komplexbildner (zum Beispiel als Eisen-Komplex oder Schutz einer phenolischen Hydroxygruppe als Methylether), Notwendigkeit mit hochgereinigten Lösungsmitteln und Apparaturen zu arbeiten.

Es besteht daher für vielfältige Zwecke ein Bedarf an stabilen, gut löslichen, und hinreichend selektiven, aber auch besser verträglichen, gut zugänglichen Komplexverbindungen, die eine möglichst große Vielfalt für eine Bindung an Makromoleküle geeigneter funktioneller Gruppen aufweisen. Der Erfindung liegt somit die Aufgabe zugrunde, diese Verbindungen und Mittel zur Verfügung zu stellen, sowie ein möglichst einfaches Verfahren zu ihrer Herstellung zu schaffen. Diese Aufgabe wird durch die Erfindung gelöst.

Es wurde gefunden, daß sich Verbindungen, die aus dem Anion einer funktionalisierten komplexbildenden Carbon- oder Phosphonsäure und einem oder mehreren Zentralion(en) eines Elements der Ordnungszahlen 21-29, 31, 32, 38, 39, 42-44 oder 57-83 sowie gegebenenfalls einem oder mehreren Kationen einer anorganischen und/oder organischen Base oder Aminosäure bestehen, überraschenderweise hervorragend zur Herstellung von NMR-, Röntgen- und Radio-Diagnostika sowie Radiotherapeutika eignen.

Die erfindungsgemäßen Verbindung werden durch die allgemeinen Formeln I und II beschrieben

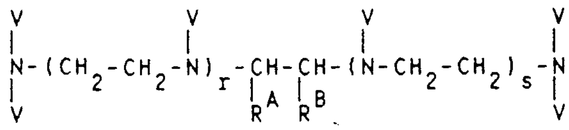

(I)

und

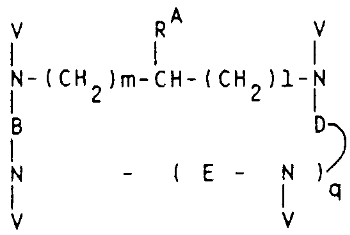

(II),

worin

q für die Ziffern 0, 1 oder 2,

r und s jeweils für die Ziffern 0, 1, 2 und 3,

m für die Ziffern 1, 2, 3, 4 oder 5,

l für die Ziffern 0, 1, 2, 3, 4 oder 5,

$R^A$ und $R^B$ jeweils für ein Wasserstoffatom oder eine gegebenenfalls Imino-, Phenylenoxy-, Phenylenimino-, Amid-, Estergruppe(n), Sauerstoff-, Schwefel-und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe, die am Ende eine funktionelle Gruppe oder gebunden über diese funktionelle Gruppe ein Makromolekül aufweist,

B, D und E, die gleich oder verschieden sind, jeweils für die Gruppe

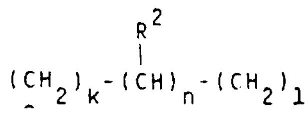

mit

$R^2$ in der Bedeutung von Wasserstoff oder einer gegebenenfalls Sauerstoff-und/oder Stickstoffatom(e) enthaltenden gegebenenfalls durch Hydroxy-und/oder Aminogruppe(n) substituierten geradkettigen, verzweigten, gesättigten oder ungesättigten $C_1$-$C_{20}$-Alkylgruppe

k in der Bedeutung der Ziffern 0, 1, 2, 3, 4 oder 5,

n in der Bedeutung der Ziffern 0 oder 1,

mit der Maßgabe, daß r und s zusammen nicht mehr als 4 ergeben und daß B, D und E jeweils mindestens 2 und maximal 5 Kohlenstoffatom(e) enthält,

V für einen Rest

a) der allgemeinen Formel IA

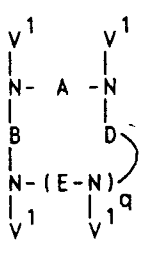

(IA),

worin

A für die Gruppe

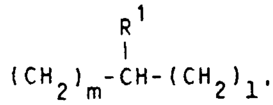

wobei $R^1$ eine gegebenenfalls Imino-, Phenylenoxy-, Phenylenimino-, Amid-, Estergruppe(n), Sauerstoff-, Schwefel- und/oder Stickstoff-Atom(e) enthaltende gegebenenfalls durch Hydroxy-, Mercapto-,Imino- und/ oder Aminogruppe(n) substituierte geradkettige, verzweigte, gesättigte oder ungesättigte $C_1$-$C_{20}$-Alkylengruppe bedeutet,

$V^1$ für V oder eine $CH_2X$-Gruppe,
wobei X die Reste -COOY oder -$PO_3HY$ mit Y in der Bedeutung eines Wasserstoffatoms und/oder eines Metallionenäquivalents eines Elements de Ordnungszahlen 21-29, 31, 32, 38, 39, 42-44 oder 57-83 bedeutet,
oder

b) der allgemeinen Formel IB oder IC

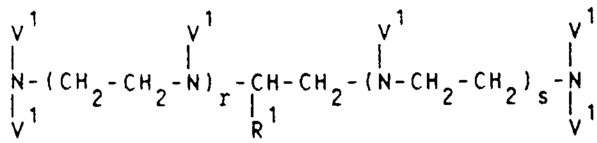 (IB),

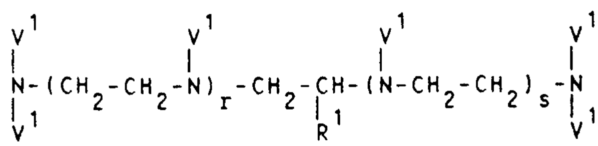 (IC),

stehen,
mit der Maßgabe, daß pro Molekül 16 bis 5000 $CH_2X$-Gruppen enthalten sind, sowie deren Salze mit anorganischen und/oder organischen Basen oder Aminosäuren.

Die Verknüpfung der Teilstrukturen IA, IB oder IC mit den Stickstoffatomen des Grundgerüstes (I) bzw. (II) erfolgt über $R^1$.

Verbindungen der allgemeinen Formel I mit Y in der Bedeutung von Wasserstoff werden als Komplexbildner und mit mindestens zwei der Substituenten Y in der Bedeutung eines Metallionenäquivalents als Metallkomplexe bezeichnet.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan (II)-, Eisen(II)-, Cobalt(II)-, Nickel(II)-, Kupfer(II)-, Praseodym(III)-, Neodym(III)-, Samarium(III)-und Ytterbium(III)-ion. Wegen ihres sehr starken magnetischen Moments sind besonders bevorzugt das Gadolinium(III)-, Terbium (III)-, Dysprosium(III)-, Holmium(III)-, Erbium(III)- und Eisen (III)-ion.

Für die Verwendung der erfindungsgemäßen Mittel in der nuklearmedizinischen Diagnostik muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Sowohl die erfindungsgemäßen Mittel, die zur Anwendung in der NMR-Diagnostik bestimmt sind, als auch jene, die für die Röntgen-Diagnostik Verwendung finden, eignen sich zur Anwendung in der Ultraschall-Diagnostik.

Die in $R^1$, $R^A$ bzw. $R^B$ enthaltene Alkylengruppe sowie die $R^2$-Alkylgruppe können geradkettig, verzweigt, cyclisch, aliphatisch, aromatisch oder arylaliphatisch sein und bis zu 20 Kohlenstoffatome aufweisen. Bevorzugt sind geradkettige $C_1$-$C_6$-gruppen sowie $C_1$-$C_4$-Alkylenphenylgruppen. Enthält die Gruppe einen Phenoxy-Baustein, so ist dieser bevorzugt p-ständig über eine Methylengruppe an die -CH-Gruppe des Grundgerüstes des Verbindung der allgemeinen Formel I gebunden.

Bevorzugte funktionelle Gruppen, die sich am Ende der $R^A$ bzw. $R^B$-Alkylengruppe befinden, sind beispielsweise die Benzylester-, Ethylester-, t-Butylester-, Amino-, $C_1$-$C_6$-Alkylamino-, Aminocarbonyl-, Hydrazino-, Hydrazinocarbonyl-, Maleimido-, Methacrylamido-, Methacryloylhydrazinocarbonyl-, Maleimidamidocarbonyl-, Halogeno-, Mercapto-, Hydrazinotrimethylenhydrazinocarbonyl-, Aminodimethylenamidocarbonyl-, Bromcarbonyl-, Phenylendiazonium-, Isothiocyanat-, Semicarbazid-, Thiosemicarbazid-, Hydroxy-Gruppe.

Zur Verdeutlichung seien einige ausgewählte Substituenten aufgeführt:

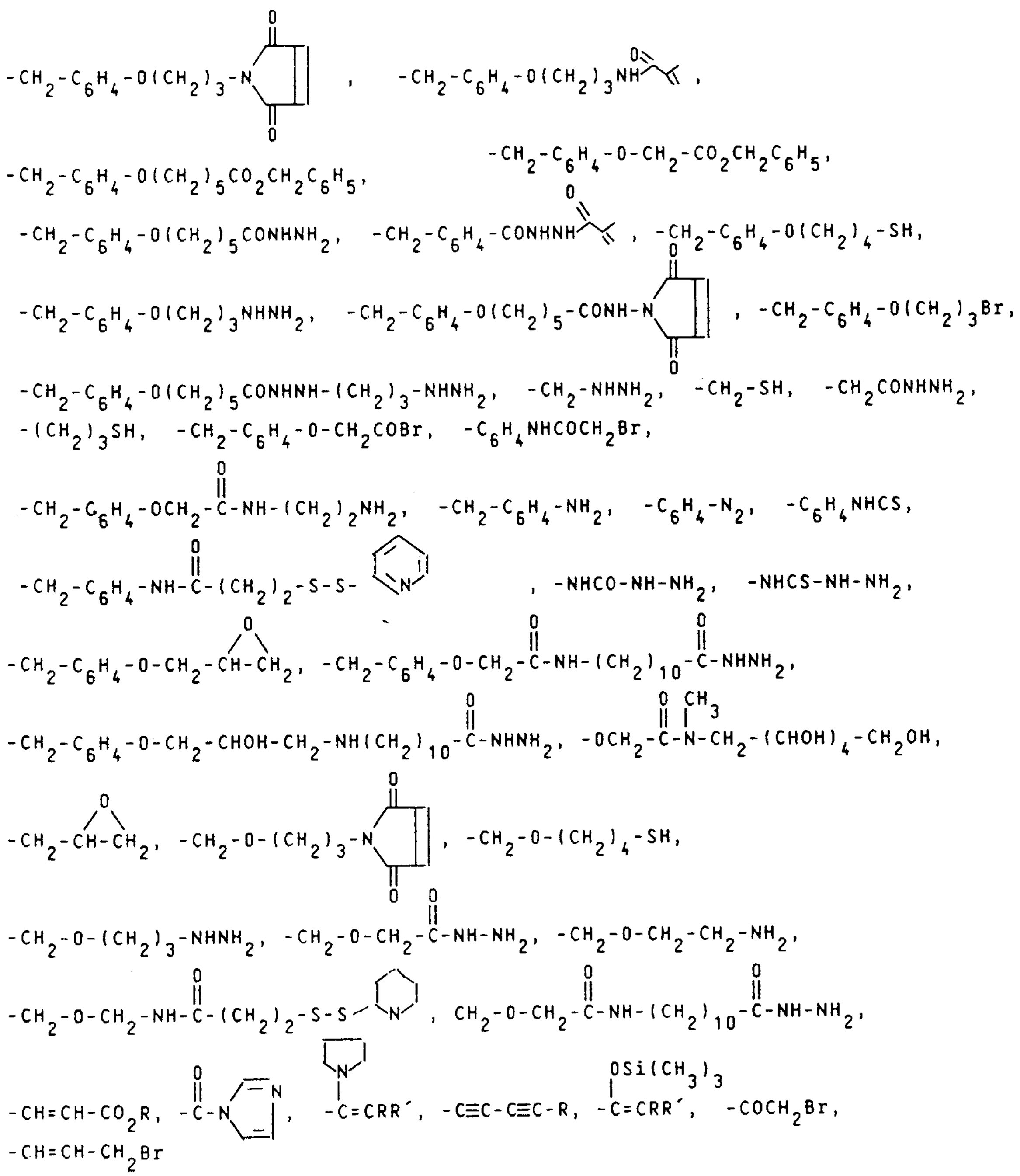

wobei R und R′ gleich oder verschieden sind und jeweils ein Wasserstoffatom, einen gesättigten oder

ungesättigten gegebenenfalls durch eine Phenylgruppe substituierten $C_1$-$C_{20}$-Alkylrest oder eine Phenylgruppe bedeuten.

Wenn nicht alle aziden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Die Herstellung der erfindungsgemäßen Komplexbildner (Y = Wasserstoff) erfolgt dadurch, daß man in an sich bekannter Weise Verbindungen der allgemeinen Formel III oder IV

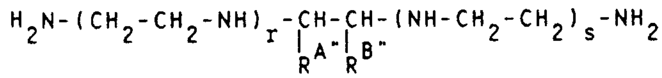

(III),

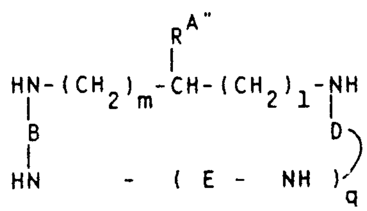

(IV),

worin r, s, m, l, q, B, D und E die oben genannte Bedeutung haben und

$R^{A''}$ und $R^{B''}$ jeweils für ein Wasserstoffatom oder einen Substituenten, der in $R^{A'}$ bzw. $R^{B'}$ umgewandelt werden kann,

stehen, mit der Maßgabe, daß r und s zusammen nicht mehr als 4 ergeben, mit einem den Rest V einführenden Reaktionspartner umsetzt,

die so, gegebenenfalls nach Abspaltung von Säureschutzgruppen $Y'$, erhaltenen Säuren der allgemeinen Formel I mit Y in der Bedeutung eines Wasserstoffatoms gewünschtenfalls

a) in an sich bekannter Weise mit mindestens einem Metalloxid oder Metallsalz eines Elements der Ordnungszahlen 21-29, 31, 32, 38, 39,42-44, 49 oder 57-83 umsetzt und anschließend, falls gewünscht, vorhandene azide Wasserstoffatome durch Kationen anorganischer und/oder organischer Basen oder Aminosäuren substituiert,

oder

b) in an sich bekannter Weise mit mindestens einem Metalloxid oder Metallsalz eines Elements der Ordnungszahlen 21-29, 31, 32, 38, 39, 42-44, 49, oder 57-83 umsetzt und anschließend die so erhaltenen Metallkomplexe in an sich bekannter Weise über die in $R^{A'}$ bzw. $R^{B'}$ enthaltene funktionelle Gruppe an ein Makromolekül bindet und, falls gewünscht, vorhandene azide Wasserstoffatome durch Kationen anorganischer und/oder organischer Basen oder Aminosäuren substituiert,

oder

c) in an sich bekannter Weise über die in $R^{A'}$ bzw. $R^{B'}$ enthaltene funktionelle Gruppe an ein Makromolekül bindet und anschließend in an sich bekannter Weise mit mindestens einem Metalloxid oder Metallsalz eines Elements der Ordnungszahlen 21-29, 31, 32, 38, 39, 42-44, 49 oder 57-83 umsetzt und anschließend, falls gewünscht, vorhandene azide Wasserstoffatome durch Kationen anorganischer und/oder organischer Basen oder Aminosäuren substituiert.

Die zur Einführung der Substituenten V vorgenommene Alkylierung der Amine der allgemeinen Formel III und IV wird mit Reagenzien durchgeführt, die diesen Substituenten (gebunden an eine Fluchtgruppe) enthalten oder aus denen der gewünschte Substituent, gegebenenfalls nach Modifikation durch Folgereaktion(en), durch die Alkylierung generiert wird. Als Beispiele für die erstgenannten seien V-Halogenide, V-mesylate und V-tosylate erwähnt.

Zur zweiten Gruppe gehören zum Beispiel Oxirane, Thiirane, Azirane, $\alpha,\beta$-ungesättigte Carbonylverbindungen oder deren Vinyloge, Aldehyde, Ketone, Isothiocyanate und Isocyanate.

Als Beispiele für Folgereaktionen seien Hydrierungen, Veresterungen, Veretherungen und Alkylierungen

genannt, die nach dem Fachmann bekannten Literaturverfahren durchgeführt werden.

Als Säureschutzgruppen $Y'$ kommen niedere Alkyl-, Aryl- und Aralkylgruppen, beispielsweise die Methyl-, Ethyl-, Propyl-,Butyl-, Phenyl-, Benzyl-,Diphenylmethyl-, Triphenylmethyl-, bis(P-Nitrophenyl)-methylgruppe, sowie Trialkylsilylgruppen in Frage.
Die Abspaltung der Schutzgruppen $Y'$ erfolgt nach den dem Fachmann bekannten Verfahren, beispielsweise durch Hydrolyse, alkalische Verseifung der Ester mit Alkali in wäßrig-alkoholischer Lösung bei Temperaturen von 0 bis 50 °C oder im Fall von tert.-Butylestern mit Hilfe von Trifluoressigsäure.

Zur Synthese der erfindungsgemäßen phosphonsäuregruppen-tragenden Verbindungen der allgemeinen Formel I und II werden die Amine der allgemeinen Formel III und IV nach dem Fachmann bekannten Verfahren (Phosphorous and Sulfur 1983, Vol. 16, 233) mit Formaldehyd/Phosphoriger Säure umgesetzt.

Die Alkylierung kann auch mit Reagenzien erfolgen, die geschützte Stickstoffatome enthalten. Nach Abspaltung dieser Schutzgruppen kann eine weitere Stickstoff-Alkylierung durchgeführt werden. Man kann diese Reaktionsfolge, die eine exponentiell anwachsende Anzahl von N-$CH_2$X-Gruppen im Molekül schafft, so oft wiederholen, bis schließlich (nach Abbruch dieser Reaktionskette durch Umsetzung mit einem Alkylierungsmittel, dessen Stickstoffatome $CH_2$X-Gruppen, gegebenenfalls in geschützter Form, tragen) insgesamt bis zu 5000 $CH_2$X-Gruppen im Molekül vorhanden sind.
Als N-Schutzgruppe ist beispielsweise die Trifluoracetylgruppe geeignet, die nach literaturbekannten Methoden (z.B. mit Natriumborhydrid) abgespalten werden kann.

Die Herstellung der als Ausgangssubstanzen benötigten Amine der allgemeinen Formel III erfolgt analog literaturbekannten Methoden (zum Beispiel Canad. Patent No. 1.178.951, Eur. I. Med. Chem.-Chim. Ther. 1985, 20,509 und 1986, 21, 333), indem man von Aminosäuren ausgeht, die in gegebenenfalls ethylenaminsubstituierte [zum Beispiel mit N-(2-Aminoethyl)-carbaminsäurebenzylester] Amide überführt und anschließend zu den gewünschten Aminen (vorzugsweise mit Diboran oder Lithiumaluminiumhydrid) reduziert werden.

Will man Verbindungen der allgemeinen Formel I mit $R^A$ in dere Bedeutung eines Wasserstoffatoms synthetisieren, so ist es notwendig, vor der Reduktion ein derartiges Amid durch Umsetzung mit zum Beispiel Ethyloxamat in einem polaren Lösungsmittel wie zum Beispiel Tetrahydrofuran, Dimethylsulfoxid oder Dimethoxyethan bei einer Temperatur zwischen 50 °C und 250 °C, vorzugsweise 70 °C bis 150 °C (gegebenenfalls in einem Druckbehälter) an der $\alpha$-Aminogruppe zu substituieren, so daß man ein 3-Aza-2-oxo-glutarsäurediamid-Derivat als Zwischenprodukt erhält.

Die Herstellung der zur Einführung der Substituenten V benötigten Reagenzien erfolgt durch Alkylierung von monosubstituierten Aminen der allgemeinen Formel Va, Vb, Vc.

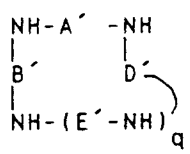

(Va),

wobei $A'$ für die Gruppe

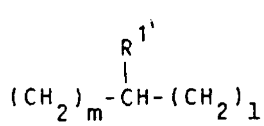

steht und $B'$, $D'$ und $E'$ die gleiche Bedeutung wie B, D und E haben, wobei allerdings gegebenenfalls in B, D und E enthaltene funktionelle Gruppen gegebenenfalls geschützt sind.

$$H_2N-(CH_2-CH_2-NH)_r-\underset{R^{1'}}{\underset{|}{C}}H-CH_2-(NH-CH_2-CH_2)_s-NH_2 \qquad (Vb),$$

$$H_2N-(CH_2-CH_2-NH)_r-CH_2-\underset{R^{1'}}{\underset{|}{C}}H-(NH-CH_2-CH_2)_s-NH_2 \qquad (Vc),$$

wobei $R^{1'}$ für einen Substituenten steht, der bei der Alkylierung der Amine der allgemeinen Formel III bzw. IV in den Substituenten $R^1$, der Va, Vb oder Vc mit den Stickstoffatomen von III bzw. IV verknüpft, umgewandelt wird, mit einem Ester der allgemeinen Formel VI

$HalCH_2COOY'$ (VI),

worin Hal für Chlor, Brom oder Jod und $Y'$ für eine Säureschutzgruppe steht. Die Umsetzung erfolgt in polaren aprotischen Lösungsmitteln wie zum Beispiel Dimethylformamid, Dimethylsulfoxid oder Hexamethylphosphorsäuretriamid in Gegenwart eines Säurefängers, wie zum Beispiel tertiäres Amin (zum Beispiel Triäthylamin, Trimethylamin, N,N-Dimethylaminopyridin, 1,5 Diazabicyclo[4.3.0] nonen-5[DBN], 1,5 Diazabicyclo [5.4.0] undecen-5(DBU), Alkali-, Erdalkalicarbonat oder -hydrogencarbonat (zum Beispiel Natrium-, Magnesium-, Calcium-, Barium-, Kalium- carbonat und -hydrogen-carbonat) bei Temperaturen zwischen -10 °C und 120 °C, vorzugsweise zwischen 0 °C und 50 °C.

Die Herstellung der cyclischen Amine der Formel IV bzw. Va erfolgt durch Cyclisierung zweier Reaktanten, von denen der eine $R^{A''}$ bzw. $R^{1''}$ -substituiert ist, wobei $R^{1''}$ für $R^{1'}$ oder einen Substituenten, der in $R^{1'}$ umgewandelt werden kann, steht.

Die Cyclisierung wird nach literaturbekannten Methoden, (zum Beispiel Org. Synth. 58,86 (1978), Makrocyclic Polyether Syntheses, Springer Verlag Berlin, Heidelberg, New York 1982, Coord.Chem.Rev. 3,3 (1968), Ann. Chem. 1976,916) durchgeführt: einer der beiden Reaktanten trägt am Kettenende zwei Fluchtgruppen, der andere zwei Stickstoffatome, die nukleophil diese Fluchtgruppen verdrängen. Als Beispiel seien genannt die Umsetzung von endständigen, gegebenenfalls ein oder zwei Stickstoffatom(e) enthaltenden, Dibrom-, Dimesyloxy-, Ditosyloxy- oder Dialkoxycarbonylalkylenverbindungen mit endständigen, gegebenenfalls ein oder zwei zusätzliche Stickstoffatom(e) in der Alkylenkette enthaltenden Diazaalkylenverbindungen, von denen einer der beiden Reaktanten $R^{A''}$ bzw. $R^{1''}$-substituiert ist.

Die Stickstoffatome sind gegebenenfalls geschützt, zum Beispiel als Tosylate, und werden vor der nachfolgenden Alkylierungsreaktion nach literaturbekannten Verfahren freigesetzt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.

Als Substituenten $R^{A''}$ bzw. $R^{B''}$, die in die am Ende eine für eine Bindung an ein Makromolekül geeignete funktionelle Gruppe aufweisenden Substituenten $R^{A'}$ bzw. $R^{B'}$ überführt werden können, sind unter anderem Hydroxy- und Nitrobenzyl-, Hydroxy-und Carboxyalkyl- sowie Thioalkylreste mit bis zu 10 Kohlenstoffatomen geeignet. Sie werden nach dem Fachmann bekannten Literaturverfahren (Chem.-Pharm. Bull. 33,674 (1985), Compendium of Org. Synthesis Vol. 1-5, Wiley and Sons, Inc.) in die gewünschten Substituenten ( zum Beispiel mit der Amino-, Hydrazino-, Hydrazinocarbonyl-, Methacryloylhydrazinocarbonyl-, Maleimidamidocarbonyl-, Halogeno-, Halogenocarbonyl-, Mercaptogruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.

Beispiele für die Umwandlung von an aromatische oder aliphatische Reste gebundenen Hydroxy- oder Aminogruppen sind die in wasserfreien, aprotischen Lösungsmitteln wie Tetrahydrofuran, Dimethoxyethan oder Dimethylsulfoxid in Gegenwart eines Säurefängers wie zum Beispiel Natriumhydroxid, Natriumhydrid oder Alkali-oder Erdalkalicarbonaten wie zum Beispiel Natrium-, Magnesium-, Kalium-, Calciumcarbonat bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel VII

Z-L-Fu (VII),

worin Z für ein Nucleofug wie z.B. Cl, Br, J. $CH_3C_6H_4SO_3$, oder $CF_3SO_3$, L für einen aliphatischen,

aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe stehen.

Als Beispiele für Verbindungen der allgemeinen Formel VII seien genannt $Br(CH_2)_2NH_2$, $Br(CH_2)_3OH$, $BrCH_2COOCH_3$, $BrCH_2CO_2{}^tBu$, $Br(CH_2)_4CO_2C_2H_5$, $BrCH_2COBr$, $BrCH_2CONH_2$, $ClCH_2COOC_2H_5$, $BrCH_2CONHNH_2$,

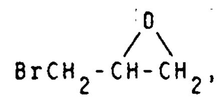

$CF_3SO_3(CH_2)_3Br$, $BrCH_2C{\equiv}CH$, $BrCH_2CH=CH_2$.

Umwandlungen von Carboxy-Gruppen können zum Beispiel nach der Carbodiimid-Methode (Fieser, Reagents for Organic Syntheses 10,142) über ein gemischtes Anhydrid [Org. Prep. Proc. Int. 7,215(1975)] oder über einen aktivierten Ester (Adv. Org. Chem. Part B, 472) durchgeführt werden.

Die Herstellung der als Ausgangssubstanzen für die Cyclisierung benötigten Amine erfolgt analog literaturbekannten Methoden.

Ausgehend von einer N-geschützten Aminosäure erhält man durch Umsetzung mit einem partiell geschützten Diamin (zum Beispiel nach der Carbodiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21,333 (1986)) mit der doppelten molaren Menge einer N-geschützten ω-Aminosäure liefert ein Tetramin nach geeigneter Aufarbeitung.

In beiden Fällen ist die Anzahl der Kohlenstoffatome zwischen den N-Atomen durch die Art der als Kopplungspartner eingesetzten Diamine bzw. Aminosäuren bestimmbar.

Die Herstellung der als Edukte für die Alkylierungsreagenzien benötigten Amine der allgemeinen Formel Vb und Vc erfolgt nach den bereits für die Herstellung der Amine III beschriebenen Methoden.

Die so erhaltenen komplexbildenden Liganden (sowie die Komplexe) können auch an Makromoleküle geknüpft sein, von denen bekannt ist, daß sie sich in dem zu untersuchenden Organ oder Organteil besonders anreichern. Solche Makromoleküle sind beispielsweise Enzyme, Hormone, Zucker, Dextrane, Lektine, Porphyrine, Bleomycine, Insulin, Prostaglandine, Steroidhormone, Aminozucker, Aminosäuren, Peptide wie Polylysin, Proteine (wie zum Beispiel Immunoglobuline und monoklonale Antikörper) oder Lipide (auch in Form von Liposomen). Besonders hervorzuheben sind Konjugate mit Albuminen, wie Humanserumalbumin, Antikörpern, wie zum Beispiel monoklonale für tumorassoziierte Antigene spezifische Antikörper, Antimyosin oder Cholsäure. Anstelle von Biomolekülen können auch geeignete synthetische Polymere wie Polyethylenimine angeknüpft werden. Die hieraus gebildeten pharmazeutischen Mittel eignen sich beispielsweise zur Anwendung in der Tumor- und Infarkt-Diagnostik sowie Tumortherapie. Als monoklonale Antikörper (zum Beispiel Nature 256, 495, 1975), die gegenüber den polyklonalen Antikörpern die Vorzüge haben, daß sie spezifisch für eine antigene Determinante sind, eine definierte Bindungsaffinität besitzen, homogen sind (damit wird ihre Reindarstellung wesentlich einfacher) und die in Zellkulturen in großen Mengen herstellbar sind, kommen für die Konjugation insbesondere solche infrage, die gegen überwiegend zellmembranständige Antigene gerichtet sind. Als solche sind zum Beispiel für die Tumordarstellung monoklonale Antikörper bzw. deren Fragmente Fab und $F(ab')_2$ geeignet, die zum Beispiel spezifisch sind für humane Tumore des Gastrointestinaltraktes, der Brust, der Leber, der Blase, der Keimdrüsen und von Melanomen (Cancer Treatment Repts. 68, 317, 1984, Bio Sci 34, 150, 1984) oder gegen Carcinoembryonales Antigen (CEA), Humanes Choriogonadotropin (β-HCG) oder andere tumorständige Antigene, wie Glycoproteine, gerichtet sind. (New Engl. J. Med. 298, 1384, 1973, US-P 4,331,647). Geeignet sind unter anderem auch Anti-Myosin, Anti-Insulin-und Anti-Fibrin-Antikörper (US-P 4,036,945).

Für Leberuntersuchungen beziehungsweise für die Tumordiagnostik eignen sich beispielsweise Konjugate oder Einschlußverbindungen mit Liposomen (die beispielsweise als unilamellare oder multilamellare Phosphatidylcholin-Cholesterol-Vesikel eingesetzt werden).

Die nach dem Stand der Technik bekannten Bindungen von zum Beispiel Radioisotopen an Immunglobuline und deren Fragmente sind mit dem Nachteil mangelnder Stabilität der markierten Antikörper-Konjugate bzw. mangelnder Spezifität (zum Beispiel infolge der Verwendung eines Diethylentriaminpentaessigsäure = DTPA-Anhydrids) behaftet (zum Beispiel Diagnostic Imaging 84, 56; Science 220, 613, 1983; Cancer Drug Delivery 1, 125, 1984).

Die Konjugatbildung gemäß vorliegender Erfindung erfolgt dagegen über die am Ende der $C_1$-$C_{20}$-Alkylengruppe des Substituenten $R^A$ bzw. $R^B$ sich befindliche funktionelle Gruppe, wie sie weiter oben

definiert ist. Es können bei der Konjugatbildung der Säuren mit Makromolekülen mehrere Säurereste an dieses gebunden werden. In diesem Fall kann jeder Säurerest ein Zentralion tragen.

Die Kopplung an die gewünschten Makromoleküle erfolgt ebenfalls nach an sich bekannten Methoden, wie sie zum Beispiel in Rev. Roum Morphol. Embryol. Physio., Physiologie 1981, 18, 241 und J. Pharm. Sci. 68, 79 (1979) beschrieben sind, beispielsweise durch Reaktion der nucleophilen Gruppe eines Makromoleküls, wie der Amino-, Phenol-, Sulfhydryl-, Aldehyd- oder Imidazol-Gruppe mit einem aktivierten Derivat des Komplexbildners. Als aktivierte Derivate kommen beispielsweise Monoanhydride, Säurechloride, Säurehydrazide, gemischte Anhydride (siehe zum Beispiel G.E. Krejcarek und K.L. Tucker, Biochem., Biophys. Res. Commun. 1977, 581), aktivierte Ester, Nitrene oder Isothiocyanate in Betracht. Umgekehrt ist es auch möglich, ein aktiviertes Makromolekül mit der komplexbildenden Säure umzusetzen. Zur Konjugation mit Proteinen bieten sich auch Substituenten zum Beispiel der Struktur $C_6H_4N_2$, $C_6,H_4NHCOCH_2$, $C_6H_4NHCS$ oder $C_6H_4OCH_2CO$ an.

Im Falle der Antikörper-Konjugate darf die Bindung des Antikörpers an den Komplexbildner (bzw. an den Metallkomplex; die Herstellung des Metall-Komplex-Konjugats kann sowohl in der Reihenfolge Komplexbildner, Komplexbildner-Konjugat, Endprodukt als auch in der Reihenfolge Komplexbildner, Metall-Komplex, Endprodukt erfolgen) nicht zum Verlust oder zur Verminderung der Bindungsaffinität und Bindungsspezifität des Antikörpers zum Antigen führen. Dies kann entweder durch Bindung an den Kohlenhydrat-Anteil im Fc-Teil des Glycoproteins bzw. in den Fab oder $F(ab')_2$-Fragmenten oder durch Bindung an Schwefelatome des Antikörpers bzw. der Antikörper-Fragmente erfolgen.

Im ersten Fall muß zunächst eine oxidative Spaltung von Zuckereinheiten zur Generation kopplungsfähiger Formylgruppen durchgeführt werden. Diese Oxidation kann auf chemischem Wege mit Oxidationsmitteln wie zum Beispiel Perjodsäure, Natriummetaperjodat oder Kaliummetaperjodat nach literaturbekannten Methoden (zum Beispiel J. Histochem and Cytochem. 22, 1084, 1974) in wäßriger Lösung in Konzentrationen von 1 bis 100, vorzugsweise 1 bis 20 mg/ml, und einer Konzentration des Oxidationsmittels zwischen 0,001 bis 10 mMol, vorzugsweise 1 bis 10 mMol in einem pH-Bereich von ca. 4 bis 8 bei einer Temperatur zwischen 0 bis 37°C und einer Reaktionsdauer zwischen 15 Minuten und 24 Stunden vorgenommen werden. Auch auf enzymatischem Wege kann die Oxidation, beispielsweise mit Hilfe von Galaktoseoxidase in einer Enzymkonzentration von 10-100 Einheiten/ml, einer Substratkonzentration von 1 bis 20 mg/ml, bei einem pH-Wert von 5 bis 8, einer Reaktionsdauer von 1 bis 8 Stunden und einer Temperatur zwischen 20 und 40 °C, durchgeführt werden (zum Beispiel J. Biol. Chem. 234, 445, 1959).

An die durch Oxidation generierten Aldehyde werden Komplexbildner (oder Metallkomplexe, siehe oben) mit geeigneten funktionellen Gruppen wie zum Beispiel Hydrazin, Hydrazid, primäres Amin, Hydroxylamin, Phenylhydrazin, Semicarbazid und Thiosemicarbazid durch Reaktion zwischen 0 - 37 °C, bei einer Reaktionsdauer von 1 bis 65 Stunden, einem pH-Wert zwischen ca. 5,5 und 8, einer Antikörperkonzentration von 0,5 bis 20 mg/ml und einem molaren Verhältnis des Komplexbildners zum Antikörperaldehyden von 1:1 bis 1000:1 gebunden. Die anschließende Stabilisierung des Konjugates erfolgt durch Reduktion der Doppelbindung z.B. mit Natriumborhydrid oder Natriumcyanoborhydrid; das Reduktionsmittel wird dabei in einem 10 bis 100fachen Überschuß verwendet (zum Beispiel J. Biol. Chem. 254, 4359, 1979).

Die zweite Möglichkeit der Bildung von Antikörper-Konjugaten geht aus von einer schonenden Reduktion der Disulfid-Brücken des Immunoglobulin-Moleküls; hierbei werden die empfindlicheren Disulfid-Brücken der H-Ketten des Antikörper-Moleküls gespalten, während die S-S-Bindungen der Antigen-bindenden Region intakt bleiben, so daß praktisch keine Verminderung der Bindungsaffinität und - spezifität des Antikörpers eintritt (Biochem. 18, 2226, 1979, Handbook of Experimental Immunology, Vol. 1, Second Edition, Blackwell Scientific Publications. London 1973, Chapter 10). Dieser freien Sulfhydryl-Gruppen der intra-H-Ketten Regionen werden dann mit geeigneten funktionellen Grupen von Komplexbildnern oder Metallkomplexen bei 0 bis 37 °C, einem pH-Wert von ca. 4 bis 7, und einer Reaktionsdauer von 3 bis 72 Stunden unter Ausbildung einer kovalenten Bindung, die die Antigen-Bindungsregion des Antikörpers nicht beeinflußt, umgesetzt. Als geeignete reaktive Gruppen seien beispielsweise genannt: Halogenalkyl-, Halogenacetyl-, p-Mercuribenzoatgruppen sowie Gruppen, die einer Michael-Additions-Reaktion, wie zum Beispiel Maleinimide, Methacrylogruppen (zum Beispiel J. Amer. Chem. Soc. 101, 3097, 1979), zu unterwerfen sind.

Es können auch Bindungen nichtkovalenter Art zur Kopplung genutzt werden, wobei sowohl ionische als auch van der Waals- und Wasserstoffbrücken-Bindungen in wechselnden Anteilen und Stärke (Schlüssel-Schloß-Prinzip) zur Bindung beitragen können (zum Beispiel Avidin-Biotin, Antikörper-Antigen). Auch Einschlußverbindungen (host-guest) kleinerer Komplexe in größere Cavitäten beim Makromolekül sind möglich.

Eine zur Herstellung von Konjugaten von sowohl Antikörper- als auch Antikörperfragmenten besonders gut geeignete Methode ist die Kopplung an einer Festphase. Hierbei wird der Antikörper oder das entsprechende $F(ab)_2$-Fragment an eine stationäre Phase (zum Beispiel einen Ionenaustauscher) gebunden,

die sich in einer temperierbaren mit Zu- und Abfluß versehenen Säule befindet. Zur Oxidation im Fc-Teil des Antikörpers muß die Säule durch Umhüllung vor Lichteinfall geschützt werden; zur Reduktion von Disulfidbrücken (zum Beispiel bei der Generierung von Fab-Fragmenten) muß unter Argon als Schutzgas gearbeitet werden können. Der eigentliche Kopplungsvorgang verläuft dann wie folgt:
Nach Spülen der Säule mit einem geeigneten Puffer wird als Eluent eine Lösung eingesetzt, die reaktive Gruppen am gebundenen Protein erzeugt (zum Beispiel Perjodat-Lösung zur Erzeugung von Aldehyd-Gruppen im Fc-Teil von monoklonalen Antikörpern oder Mercaptoethylamin-Lösung zur Herstellung von Sulfhydrylgruppen in Fragmenten). Nachdem die Reaktionslösung den vorherigen Eluenten vollständig verdrängt hat, stoppt man den Durchfluß für eine zur vollständigen Umsetzung ausreichende Zeit, spült anschließend ausreichend mit Puffer, zieht dann eine Lösung mit dem Kopplungspartner (zum Beispiel dem Hydrazid oder Dithiopyridyl Deivat eines Komplexbildners oder eines Komplexes) auf und stoppt den Durchfluß wieder ausreichend lange. Statt den Durchfluß für längere Zeit zu stoppen, kann man auch eine sogenannte recycle-Schaltung verwenden; hierbei wird das die Säule verlassende Eluat mittels einer Schleifenschaltung direkt wieder auf die Säule gepumpt. Man erzielt hierbei wegen der besseren Durchmischung wesentlich kürzere Reaktionszeiten und bessere Ausbeuten. Danach folgt wieder eine Spülung mit Puffer-Lösung. Ist ein freier Komplexbildner der Kopplungspartner, wird in einem weiteren Zyclus mit einer Lösung des gewünschten Metallsalzes (zum Beispiel einer Citratlösung) sowie anschließendem Spülgang komplexiert. Schließlich eluiert man das Konjugat mit einem pH- oder Salzgradienten. Anschließend wird, gegebenenfalls nach Entsalzen, lyophilisiert. Nach Äquilibrieren mit Pufferlösung ist die Säule den nächsten Kopplungsgang bereit.
Diese Methode ist sowohl zur Darstellung sehr kleiner als auch sehr großer Mengen an Konjugat den herkömmlichen Verfahren sowohl in Geschwindigkeit als auch an Ausbeute weit überlegen und erlaubt auch die kontinuierliche Herstellung von Konjugaten; dies ist die Voraussetzung für eine wirtschaftliche Produktion größerer Mengen.

Die so gebildeten Verbindungen werden anschließend vorzugsweise chromatographisch über Ionenaustauscher auf einer Fast-Protein-Liquid-Chromatography-Anlage gereinigt.
Die so erhaltenen Verbindungen der allgemeinen Formel I mit Y in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten Y in der Bedeutung eines Metallionenäquivalents überführt werden.

Die Herstellung der erfindungsgemäßen Metallkomplexe erfolgt in der Weise, wie sie in der Patentschrift DE-OS 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 38, 39, 42-44, 49, 57-83 in Wasser und/oder einem niederen Alkohol (wie Methanol, Ethanol oder Isopropanol) löst oder suspendiert und mit der Lösung oder Suspension der äquivalenten Menge der komplexbildenden Säure der allgemeinen Formel I mit Y in der Bedeutung eines Wasserstoffatoms umsetzt und anschließend, falls gewünscht, vorhandene azide Wasserstoffatome von Säuregruppen durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium oder Lithium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol. Ethanol, Isopropanol und anderen), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie azide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es

gewünschtenfalls reinigt und dann zur vollständigen Neutralisation mit der benötigten Menge anorganischer Base versetzt. Die Reihenfolge der Basenzugabe kann auch umgekehrt werden.

Die Konjugate aus Antikörper und Komplex werden der in-vivo Anwendung nach Inkubation mit einem schwachen Komplexbildner, wie zum Beispiel Natriumcitrat, Natrium-Ethylendiamintetraessigsäure dialysiert, um schwachgebundene Metallatome zu entfernen.

Im Falle der Verwendung von Radioisotope enthaltenden Komplexverbindungen kann deren Herstellung nach den in "Radiotracers for Medical Applications", Volume 1, CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Kom plexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween(R), Myrj(R) und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel erhalten vorzugsweise 1µMol - 1 Mol/l des Komplexsalzes und werden in der Regel in Mengen von 0,001 - 5 mMol/kg dosiert. Sie sind zur enteralen und parenteralen Applikation bestimmt.

Die erfindungsgemäßen Komplexverbindungen kommen zur Anwendung

1. für die NMR-, Röntgen- und Ultraschall-Diagnostik in Form ihrer Komplexe mit den Ionen der Elemente mit den Ordnungszahlen 21-29, 42, 44 und 57-83;
2. für die Radiodiagnostik und Radiotherapie in Form ihrer Komplexe mit den Radioisotopen der Elemente mit den Ordnungszahlen 27, 29, 31, 32, 38, 39, 43, 49, 62, 64, 70 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es hochkonzentrierte Lösungen herzustellen, damit die Volumenbelastung des Kreislaufs in vertretbaren Grenzen zu halten und die Verdünnung durch die Korperflüssigkeit auszugleichen. Weiterhin weisen die erfindungsgemäßen Mittel nicht nur eine hohe Stabilität in-vitro auf, sondern auch eine überraschend hohe Stabilität in- vivo, so daß eine Freigabe oder ein Austausch der in den Komplexen nicht kovalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Besonders niedrige Dosierungen (unter 1 mg/kg) von organspezifischen NMR-Diagnostika sind zum Beispiel zum Nachweis von Tumoren und von Herzinfarkten einsetzbar.

Ferner können die erfindungsgemäßen Komplexverbindungen vorteilhaft als Shift-Reagenzien verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer

Anwendung und Dosierung werden z.B. in "Radiotracers for Medical Applications", CRC-Press, Boca Raton, Florida beschrieben.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$ und $^{68}Ga$ verwendet. (Heiss, W.D., Phelps, M.E., Position Emission Tomography of Brain, Springer Verlag Berlin, Heidelberg, New York 1983).

Die erfindungsgemäßen Verbindungen können auch in der Radioimmunotherapie verwendet werden. Diese unterscheidet sich von der entsprechenden Diagnostik nur durch die Menge und Art des verwendeten radioaktiven Isotops. Ziel ist dabei die Zerstörung von Tumorzellen durch energiereiche kurzwellige Strahlung mit einer möglichst geringen Reichweite. Die Spezifität des verwendeten Antikörpers ist dabei von entscheidender Bedeutung, da unspezifisch lokalisierte Antikörperkonjugate zur Zerstörung von gesundem Gewebe führen.

Der Antikörper bzw. das Antikörper-Fragment des erfindungsgemäßen Antikörper-Metall-Komplexes dient dazu, den Komplex immunspezifisch für das betreffende Antigen an das Zielorgan zu transportieren, wo das wegen seiner zelltötenden Eigenschaften ausgewählte Metallion Strahlen emittieren kann, die die Zellen lethal schädigen. Geeignete $\beta$-emittierende Ionen sind, zum Beispiel $^{46}Sc$, $^{47}Sc$, $^{48}Sc$, $^{72}Ga$ und $^{73}Ga$. Geeignete geringe Halbwertszeiten aufweisende $\alpha$-emittierende Ionen sind zum Beispiel $^{211}Bi$, $^{212}Bi$, $^{213}Bi$ und $^{214}Bi$, wobei $^{212}Bi$ bevorzugt ist.

Bei der in- vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.V. appliziert.

Details der Anwendung von Radiotherapeutika werden zum Beispiel in R.W. Kozak et al, TIBTEC, Oktober 1986, 262 diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 -5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Die erfindungsgemäßen Mittel sind - da ihre akustische Impedanz höher ist als die von Körperflüssigkeiten und Geweben - auch als Kontrastmittel für die Ultraschalldiagnostik geeignet, insbesondere in Form von Suspensionen. Sie werden im allgemeinen in Mengen von 0,1 bis 5 mMol/kg, vorzugsweise von 0,25 bis 1 mMol/kg dosiert.

Details der Anwendung von Ultraschalldiagnostika werden zum Beispiel in T.B. Tyler et al., Ultrasonic Imaging 3.323 (1981), J.I. Haft, "Clinical Echokardiography", Futura, Mount Kisco, New York 1978 und G. Stefan "Echokardiographie" G. Thieme Stuttgart/New York 1981, beschrieben.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstandes.

**BEISPIEL 1**

a) O-Benzyl-N-trifluoracetyltyrosin

112,5 g (0,41 mMol) O-Benzyltyrosin werden in 1 l trockenem Methanol suspendiert und bei Raumtemperatur mit 58,9 ml (0,42 Mol) Triethylamin versetzt. Nach Zugabe von 67 ml (0,53 Mol) Trifluoressigsäureethylester wird 130 Stunden bei Raumtemperatur unter Wasserausschluß gerührt. Man trennt von unumgesetzten Ausgangsmaterial ab und entfernt flüchtige Komponenten durch Schütteln mit Essigester/wäßriger Salzsäure. Die Essigesterphase wird mit Aktivkohle entfärbt. Nach Verdampfen der Lösungsmittel erhält man 120,7 g (80% der Theorie) farbloser Kristalle.

Schmelzpunkt: 149-150 °C

| Analyse: | | | | | |
|---|---|---|---|---|---|
| Ber.:<br>Gef.: | C 58,85<br>C 58,78 | H 4,39<br>H 4,29 | N 3,81<br>N 3,79 | O 17,42 | F 15,51<br>F 15,57 |

b) O-Benzyl-N-trifluoracetyltyrosin-(2-carbobenzoxyaminoethylen)-amid

18,5 g (50,4 mMol) O-Benzyl-N-trifluoracetyltyrosin (Beispiel 1a) werden in 200 ml trockenem Tetrahydrofuran gelöst, mit 7 ml $Et_3N$ versetzt und dann tropfenweise 4,8 ml (50,8 mMol) Chlorameisensäureethylester zugefügt, wobei die Temperatur auf unter -10 °C gehalten wird. Nach Beendigung der Zugabe wird 30 Minuten bei dieser Temperatur gerührt, nochmals mit der gleichen Menge vorgekühltem Triethylamin versetzt und eine eiskalte Lösung von 11,6 g (50,4 mMol) N-(2-Aminoethyl)-carbaminsäurebenzylester-hydrochlorid in 100 ml Dimethylformamid zugetropft. Man rührt noch 30 Minuten bei -10 °C, läßt dann unter Rühren auf Raumtemperatur kommen und erwärmt dann 10 Minuten auf 30 °C. Danach entfernt man das Lösungsmittel am Rotationsverdampfer und gießt auf 750 ml Eiswasser. Das Kristallisat wird abgesaugt, mit Eiswasser gewaschen und getrocknet. Die Ausbeute beträgt 26,9 g (94% der Theorie).

Schmelzpunkt: 189-190 °C

| Analyse: | | | | | |
|---|---|---|---|---|---|
| Ber.:<br>Gef.: | C 61,87<br>C 61,90 | H 5,19<br>H 5,08 | N 7,73<br>N 7,77 | O 14,71 | F 10.48<br>F 10.43 |

c) O-Benzyltyrosin-(2-carbobenzoxyaminoethylen)-amid

25,9 g (47,8 mMol) O-Benzyl-N-trifluoracetyltyrosin-(2-carbobenzoxyaminoethylen)-amid (Beispiel 1b) werden in 300 ml EtOH suspendiert und portionsweise mit 7,2 g (191 mMol) Natriumborhydrid versetzt. Nach Rühren über Nacht bei Raumtemperatur wird mit 50 ml Aceton versetzt und mehrmals mit Essigester extrahiert. Die organische Phase lieferte nach Trocknen und Einengen 18,8 g (88% der Theorie) weißer Kristalle vom Schmelzpunkt 145 °C.

| Analyse: | | | | |
|---|---|---|---|---|
| Ber.:<br>Gef.: | C 69,77<br>C 69,79 | H 6,53<br>H 6,53 | N 9,38<br>N 9,35 | O 14,29 |

d) Tyrosin-(2-aminoethylen)-amid

42,3 g (96,4 mMol) O-Benzyltyrosin-(2-carbobenzoxyaminoethylen)-amid (Beispiel 1c) löst man in 1,1 l Methanol, fügt 2 g 10% Palladium-Kohle zu und hydriert unter Rühren bis keine weitere Wasserstoffaufnahme mehr erfolgt. Der Katalysator wird abfiltriert und das Lösungsmittel abgedampft. Man löst in der Hitze in Methanol und fällt mit Ether: 17 g (86% der Theorie) farblose Kristalle.

Schmelzpunkt: 138-141 °C

| Analyse: | | | | |
|---|---|---|---|---|
| Ber.:<br>Gef.: | C 59,17<br>C 59,23 | H 7,67<br>H 7,51 | N 18,81<br>N 18,90 | O 14,33 |

e) 3-Aza-1-(4-hydroxybenzyl)-pentan-1,5-diamin .Trihydrochlorid

6,55 g (29,3 mMol) Tyrosin-(2-aminoethylen)-amid (Beispiel 1d) werden in 130 ml trockenem Tetrahydrofuran suspendiert und ein langsamer Strom von $B_2H_6$ (aus 5,8 g $NaBH_4$ in 75 ml Diethylenglykoldimethylether und 54 ml Bortrifluorid-Etherat-Komplex) mit trockenem Stickstoff unter stetigem Rühren durch die Lösung getrieben. Man rührt über Nacht bei 60 °C, tropft danach bei 20 °C 30 ml Methanol zu und leitet unter Eiskühlung Chlorwasserstoff ein. Man kocht danach kurz auf und saugt ab. Das Trihydrochlorid wird in Form farbloser Kristalle (8,04 g; 86% der Theorie) erhalten.

Schmelzpunkt: 250 ° C (Zersetzung)

| Analyse: | | | | | |
|---|---|---|---|---|---|
| Ber.:<br>Gef.: | C 41,45<br>C 41,37 | H 6,95<br>H 6,89 | N 13,18<br>N 13,14 | O 5,02 | Cl 33,37<br>Cl 33,51 |

f) N-Carbobenzoxyserin-(2-carbobenzoxyaminoethylen)-amid

7,34 g (30,7 mMol) N-Carbobenzoxyserin werden nach der für Beispiel 1b gegebenen Vorschrift mit den entsprechenden äquimolaren Mengen Chlorameisensäureethylester, Triethylamin und N-(2-Aminoethyl)-carbaminsäurebenzylester-Hydrochlorid umgesetzt und aufgearbeitet. Man erhält 10,33 g (81% der Theorie) farbloses Kristallisat.

Schmelzpunkt: 167 ° C

| Analyse: | | | | |
|---|---|---|---|---|
| Ber.:<br>Gef.: | C 60,71<br>C 60,75 | H 6,06<br>H 5,98 | N 10,11<br>N 10,15 | O 23,10 |

g) (2-Aminoethyl)-serinamid

13,46 g (32,4 mMol) N-Carbobenzoxyserin-(2-carbobenzoxyaminoethylen)-amid werden in 200 ml Methanol in Gegenwart von 1,37 g 10% Palladium/Kohle solange hydriert, bis kein Wasserstoff mehr aufgenommen wird. Man filtriert vom Katalysator ab und entfernt alle flüchtigen Anteile an der Ölpumpe. Es hinterbleibt ein zähes, teilweise kristallines Öl.

Ausbeute 4,67 g (98% der Theorie)

| Analyse: | | | | |
|---|---|---|---|---|
| Ber.:<br>Gef.: | C 40,80<br>C 40,71 | H 8,89<br>H 8,85 | N 28,55<br>N 28,30 | O 21,74 |

h) 1-Hydroxymethyl-1,3,5-triazapentan . Trihydrochlorid

Analog zur Vorschrift für Beispiel 1e erhält man die Titelverbindung in 67% Ausbeute aus dem vorstehend beschriebenen Amid als weißes, kristallines Pulver.

Schmelzpunkt: 236 ° C (Zersetzung)

| Analyse: | | | | | |
|---|---|---|---|---|---|
| Ber.:<br>Gef.: | C 24,75<br>C 24,71 | H 7,47<br>H 7,40 | N 17,32<br>N 17,41 | O 6,59 | Cl 43,84<br>Cl 43,75 |

i) 3-Aza-1-hydroxymethyl-1,3,5-tris-tosyl-pentan-diamin

12,32 g (50,8 mMol) des nach 1h) erhaltenen Triazapentans (als Trihydrochlorid) werden in 250 ml trockenem Pyridin vorgelegt und bei 0 ° C unter gutem Rühren 29,3 g (153,9 mMol) Tosylchlorid, gelöst in 100 ml Pyridin, über eine Stunde zugetropft. Man läßt über Nacht bei 4 ° C stehen, dampft den Hauptteil des Pyridins im Vakuum ab und nimmt in 300 ml Dichlormethan auf. Durch mehrmaliges Waschen mit verdünnter Salzsäure, wäßriger Bicarbonatlösung und bidestilliertem Wasser entfernt man restliches Pyridin und überschüssige Toluolsulfonsäure. Nach Trocknen wird an Kieselgel mit Essigester/Hexan chromatographiert. Man erhält das Tritosylat in Form farbloser Kristalle. Ausbeute 23,0 g (76% der Theorie).

Schmelzpunkt: 138-140 ° C

| Analyse: | | | | | |
|---|---|---|---|---|---|
| Ber.:<br>Gef.: | C 52,41<br>C 50,36 | H 5,58<br>H 5,82 | N 7,05<br>N 7,17 | O 18,79 | S 16,14<br>S 16,91 |

j) 2-Hydroxymethyl-1,4,7,10-tetra-tosyl-1,4,7,10-tetraazacyclododecan
15,67 g (26,3 mMol) der nach 1i) erhaltenen Verbindung werden in 150 ml Dimethylformamid gelöst und mit 1,77 g (73,65 mMol) Natriumhydrid (80% in Paraffin) 30 Minuten bei 35-40°C gerührt. Danach versetzt man langsam mit einer Lösung von 15,5 g (26,57 mMol) 3-Aza-1,3,5-tritosyl-1,5-dihydroxypentan in 150 ml DMF. Man rührt 4 Stunden bei 130°C, läßt über Nacht erkalten und destilliert das Lösungsmittel ab. Der Rückstand kristallisiert beim Verreiben mit wenig Methanol. Nach Waschen mit verdünnter Salzsäure und Umkristallisieren aus Acetonitril erhält man 11,42 g (53% der Theorie) farblose Kristalle vom Schmelzpunkt 172°C.

| Analyse: | | | | | |
|---|---|---|---|---|---|
| Ber.:<br>Gef.: | C 54,25<br>C 54,03 | H 5,66<br>H 5,68 | N 6,84<br>N 6,80 | O 17,58 | S 15,65<br>S 15,62 |

k) 2-Hydroxymethyl-1,4,7,10-tetraaza-cyclododecan . Trihydrobromid
27,6 g (32,7 mMol) der nach 1j) erhaltenen Verbindung werden in 150 ml Eisessig mit 33% Bromwasserstoff 4 Stunden auf 100°C erhitzt. Man gießt in 900 ml Diethylether, saugt ab und resuspendiert zweimal in jeweils 500 ml Diethylether. Alle Operationen werden unter Stickstoffschutzatmosphäre durchgeführt. Nach dem Trocknen im Vakuum liegen 11,61 g (77,4% der Theorie) farbloser Kristalle vor, die bei 97°C unter Zersetzung schmelzen.

| Analyse: | | | | | |
|---|---|---|---|---|---|
| Ber.:<br>Gef.: | C 24,28<br>C 24,32 | H 5,66<br>H 5,73 | N 12,59<br>N 12,37 | O 3,61 | Br 53,86<br>Br 53,92 |

l) 2-Hydroxymethyl-1,4,7,10-tetrakis-(tert.-butoxycarbonylmethyl)-1,4,7,10-tetraazacyclododecan
8,49 g (19,08 mMol) des nach 1k) erhaltenen Cyclododecans in 150 ml Dimethylformamid suspendiert, mit 12,82 g (152,6 mMol) Natriumhydrogencarbonat versetzt und bei 60°C mit einer Lösung von 15,25 g (78,2 mMol) Bromessigsäure-tert.-butylester in 100 ml DMF versetzt. Nach 2 Stunden Rühren bei dieser Temperatur wird das Lösungsmittel abgezogen und der ölige Rückstand an Kieselgel mit Ether/Hexan chromatographiert. Man erhält ein farbloses viskoses Öl. Ausbeute: 14,69 g (77% der Theorie)

| Analyse: | | | | |
|---|---|---|---|---|
| Ber.:<br>Gef.: | C 60,15<br>C 60,37 | H 9,48<br>H 9,36 | N 8,50<br>N 8,36 | O 21,85 |

m) 2-{(Oxiranylmethoxy)-methyl}-1,4,7,10-tetrakis-(tert.-butoxycarbonylmethyl)-1,4,7,10-tetraazacyclododecan
11,0 g (17,2 mMol) der Titelverbindung von1l) werden in 250 ml Toluol gelöst und mit 330 mg Natriumhydrid (80% in Paraffin, 17,2 mMol) versetzt. Man erwärmt auf 80-100°C und versetzt tropfenweise mit einer Lösung von 1,62 g (17,2 mMol) Epichlorhydrin in 50 ml Toluol. Nach zweistündigem Erhitzen auf Rückflußtemperatur wird eingeengt und über eine Kieselgelsäule gereinigt. Man erhält ein farbloses Öl. Ausbeute: 8,9 g (72% der Theorie)

| Analyse: | | | | |
|---|---|---|---|---|
| Ber.:<br>Gef.: | C 60,44<br>C 60,14 | H 9,30<br>H 9,89 | N 7,83<br>N 7,52 | O 22,37 |

n) N,N,N,N,N-Pentakis-{5-<2,5,8,11-tetraaza-2,5,8,11-tetrakis-(tert.-butoxycarbonylmethyl)-cyclododecyl>-2-hydroxy-4-oxa-pentyl}-1-(4-hydroxybenzyl)-3-aza-1,5-diaminopentan

Zu einer Suspension von 1,13 g (3,55 mMol) 3-Aza-1-(4-hydroxybenzyl)-pentan-1,5-diamin .Trihydrochlorid (Beispiel 1e) in 20 ml Methanol tropft man unter Rühren 12,75 g (17,73 mMol) der Titelverbindung 1m) in 100 ml Tetrahydrofuran, das 1,07 mg (10,64 mMol) Triethylamin enthält. Nach 2 Stunden bei Raumtemperatur erwärmt man auf 40°C und dampft die Lösung langsam ein. Die letzten Reste Methanol entfernt man im Vakuum, nimmt in Essigester auf und extrahiert mehrmals mit Wasser. Nach Trocknen über Natriumsulfat engt man ein. Es hinterbleibt ein farbloses Öl, das nach Chromatographie an Kieselgel mit Ether/Hexan mit 1 Vol% Triethylamin analysenrein erhalten wird, nachdem das Triethylamin durch Filtration über Kieselgel entfernt wurde.

Ausbeute: 8,76 g (65% der Theorie)

| Analyse: | | | | |
|---|---|---|---|---|
| Ber.:<br>Gef.: | C 60,62<br>C 60,77 | H 9,29<br>H 9,29 | N 8,51<br>N 8,48 | O 21,56 |

o) N,N,N,N,N-Pentakis-{5-<2,5,8,11-tetraaza-2,5,8,11-tetrakis-(carboxymethyl)-cyclododecyl>-2-hydroxy-4-oxa-pentyl}-1-(4-hydroxybenzyl)-3-aza-1,5-diaminopentan

2,31 g (0,61 mMol) der nach 1n) erhaltenen Verbindung werden in 30 ml Trifluoressigsäure 3 Stunden auf 40°C erwärmt und nach Abkühlen in 150 ml Ether gegossen. Der Niederschlag wird aus Tetrahydrofuran/Dichlormethan umkristallisiert. Man erhält 1,32 g (81% der Theorie) weißer Kristalle vom Schmelzpunkt 157-160 C.

| Analyse: | | | | |
|---|---|---|---|---|
| Ber.:<br>Gef.: | C 50,08<br>C 49,97 | H 7,15<br>H 7,10 | N 12,10<br>N 12,13 | O 30,65 |

Gadolinium-Komplex

Man löst 6,28 mg (2,35 mMol) des Komplexbildners in 20 ml 0,1N Ammonacetat-Lsg und versetzt mit 11,9 ml einer Lösung, die 0,9857 n an Gadoliniumacetat ist. Man erhöht den pH-Wert mit verd. Ammoniaklösung auf 7,5 und erhitzt 30 Minuten auf 80°C. Nach Zentrifugation wird der Überstand gefriergetrocknet. Man erhält 8,02 g des Komplexes (99% der Theorie) als weißes Pulver.

| Analyse.: | | | | | |
|---|---|---|---|---|---|
| Ber.:<br>Gef.: | C 38,83<br>C 38,96 | H 5,10<br>H 5,14 | N 9,38<br>N 9,45 | O 23,76 | Gd 22,90<br>Gd 22,80 |

Natrium-Salz des Gd-Komplexes

3,78 g 1,1-mMol) des Komplexes werden in 25 ml Wasser gelöst, mit 5,11 ml einer 1,073 n-Natronlauge versetzt und gefriergetrocknet. Es hinterbleiben 3,55 g (quantitativ) eines weißen Pulvers.

| Analyse: | | | | | | |
|---|---|---|---|---|---|---|
| Ber.:<br>Gef.: | C 37,39<br>C 37,19 | H 4,75<br>H 4,79 | N 9,03<br>N 8,96 | O 22,88 | Na 3,86<br>Na 3,87 | Gd 22,05<br>Gd 22,07 |

Methylglucaminsalz des Gd-Komplexes

2,0 g (0,58 mMol) des Komplexes werden zusammen mit 565 mg (2,9 mMol) N-Methylglucamin in 100 ml Wasser gelöst, filtriert und gefriergetrocknet. Es bleiben 2,63 g (98% der Theorie) farblose Kristalle zurück.

| Analyse: | | | | | |
|---|---|---|---|---|---|
| Ber.:<br>Gef.: | C 39,91<br>C 40,28 | H 6,04<br>H 5,98 | N 8,82<br>N 8,77 | O 28,14 | Gd 17,07<br>Gd 17,24 |

**BEISPIEL 2**

a) 3-Aza-2-(4-benzyloxybenzyl)-4-oxoglutarsäurediamid (Methode A)
3,62 g (13,3 mMol) O-Benzyltyrosinamid werden mit 2,7 g Ethyloxamat (23 mMol) 14 Stunden in Dimethoxyethan am Rückfluß gekocht. Nach Abziehen des Lösungsmittels wäscht man sukzessive mit Wasser, Ethanol und Ether. Nach Trocknen erhält man 2,73 g weißer Kristalle (60% der Theorie).
Schmelzpunkt: 270 ° C

| Analyse: | | | | |
|---|---|---|---|---|
| Ber.:<br>Gef.: | C 63,33<br>C 63,24 | H 5,61<br>H 5,52 | N 12,30<br>N 12,14 | O 18,74 |

oder nach Methode B
α) 3-Aza-2-(4-benzyloxybenzyl)-4-oxoglutarsäure-5-ethylester-1-amid
3 g (11,1 mMol) O-Benzyltyrosinamid werden in 30 ml Dimethoxyethan gelöst, mit 1,56 ml Triethylamin versetzt und bei 0 ° C 1,53 g (11,1 mMol) Oxalsäureethylesterchlorid zugetropft. Nach 30 Minuten bei 0 ° C gießt man auf 100 ml Eis, saugt ab und trocknet. Die Ausbeute beträgt 3,87 g (94% der Theorie)
Schmelzpunkt: 142 ° C

| Analyse: | | | | |
|---|---|---|---|---|
| Ber.:<br>Gef.: | C 64,85<br>C 64,71 | H 5,98<br>H 6,11 | N 7,56<br>N 7,46 | O 21,59 |

β) 3,6 g (9,72 mMol) 3-Aza-2-(4-benzyloxybenzyl)-1-oxoglutarsäure-5-ethylester-1-amid (Beispiel α1) werden mit 40 ml einer Lösung von 1 Mol $NH_3$/l Methanol übergossen. Nach 1 Stunde filtriert man das ausgefallene Produkt ab. Nach Trocknen werden 3,13 g (95% der Theorie) der Titelverbindung in Form farbloser Kristalle erhalten.
Schmelzpunkt: 269 ° C

| Analyse: | | | | |
|---|---|---|---|---|
| Ber.:<br>Gef.: | C 63,33<br>C 63,25 | H 5,61<br>H 5,63 | N 12,30<br>N 12,17 | O 18,74 |

b) 3-Aza-2-(4-hydroxybenzyl)-4-oxoglutarsäurediamid
1 g (2,9 mMol) 3-Aza-2-(4-benzyloxybenzyl)-4-oxoglutarsäurediamid (Beispiel a) wird mit 100 mg 10% Palladium-Kohle und einigen Tropfen konzentrierter Salzsäure in 20 ml Methanol suspendiert und bis zum Ende der Wasserstoffaufnahme hydriert. Nach Abfiltrieren vom Katalysator erhält man 690 mg farblose Kristalle (93% der Theorie).
Schmelzpunkt: 245-250 C (Zersetzung)

| Analyse: | | | | |
|---|---|---|---|---|
| Ber.:<br>Gef.: | C 52,58<br>C 52,83 | H 5,21<br>H 5,19 | N 16,72<br>N 16,84 | O 25,47 |

c) 3-Aza-2-(4-hydroxybenzyl)-pentan-1,5-diamin .Trihydrochlorid
1 g (4,0 mMol) 3-Aza-2-(4-hydroxybenzyl)-4-oxoglutarsäurediamid (Beispiel 2b) werden nach der für Beispiel 1e gegebenen Vorschrift umgesetzt. Das erhaltene farblose Kristallisat wiegt 1,19 g (93,7% der Theorie).
Schmelzpunkt: 238 ° C

| Analyse: | | | | | |
|---|---|---|---|---|---|
| Ber.:<br>Gef.: | C 41,45<br>C 41,40 | H 6,95<br>H 6,98 | N 13,18<br>N 13,07 | O 5,02 | Cl 33,37<br>Cl 33,33 |

d) 3,6,9-Triaza-3,6,9-tris-(tert.-butoxycarbonylmethyl)-4-hydroxymethyl undecandisäure-bis-(tert.-butyl)-diester
17,85 g (73,59 mMol) des nach 1h) erhaltenen Triamins werden in 450 ml Dimethylformamid vorgelegt und mit 54,4 g (648 mMol) Natriumhydrogencarbonat versetzt. Bei 35 ° C tropft man unter Rühren 78,95 g (404,72 mMol) Bromessigsäure-tert.-butylester zu, rührt danach noch 3 Stunden bei 35 ° C nach und filtriert vom ausgefallenen Salz ab. Nach Einengen versetzt man den Rückstand mit Wasser und extrahiert mehrmals mit Ether. Man trennt vom unumgesetztem Bromessigsäureester an einer Kieselgelsäule ab und erhält nach Abziehen der Lösungsmittel 42,32 g (60,12 mMol) eines farblosen zähen Öls. (81,7 % der Theorie)

| Analyse: | | | | |
|---|---|---|---|---|
| Ber.:<br>Gef.: | C 59,72<br>C 59,66 | H 9,30<br>H 9,17 | N 5,97<br>N 5,92 | O 25,0 |

e) 3,6,9-Triaza-3,6,9-tris-(tert.-butoxycarbonylmethyl)-4-[(oxiranylmethoxy) methyl]-undecandisäure-bis-(tert.-butyl)-diester
24,16 g (34,32 mMol) der nach 2d) erhaltenen Hydroxymethyl-Verbindung werden mit 1,47 g (37,76 mMol) Natriumamid in 500 ml trockenem Toluol gelöst und bei 40 ° C langsam mit einer Lösung von 3,46 g (37,41 mMol) Epichlorhydrin in 50 ml Toluol versetzt. Nach einer Stunde Rühren bei dieser Temperatur saugt man Unlösliches ab, engt ein und reinigt über eine Kieselgelsäule. Es bleiben 24,0 g (31,58 mMol) farbloses Öl zurück. (92% der Theorie)

| Analyse: | | | | |
|---|---|---|---|---|
| Ber.:<br>Gef.: | C 60,05<br>C 60,11 | H 9,15<br>H 9,32 | N 5,53<br>N 5,49 | O 25,26 |

f) 3-Aza-1,5-diamino-2-(4-hydroxybenzyl)-N,N,N,N,N-pentakis-[2-hydroxy-4-oxa-6,10-bis-<di-(tert.-butoxycarbonylmethyl)-aza>-decyl]-pentan
Wie für Beispiel 1n) beschrieben, werden 2,16 g (6,78 mMol)- 3-Aza-2-(4-hydroxybenzyl)-1,5-diaminopentan Trihydrochlorid (Beispiel 2c) mit der fünffachen molaren Menge der Titelverbindung von Beispiel 1e) umgesetzt und aufgearbeitet. Man erhält so 21,11 g (71% der Theorie) der Titelverbindung als farbloses Öl.

| Analyse: | | | | |
|---|---|---|---|---|
| Ber.:<br>Gef.: | C 60,21<br>C 60,50 | H 9,15<br>H 9,19 | N 6,28<br>N 6,26 | O 24,34 |

g) 3-Aza-1,5-diamino-2-(4-hydroxybenzyl)-N,N,N,N,N-pentakis-[2-hydroxy-4-oxa-6,10-bis-[di-(carboxymethyl)-amino]-8-<(carboxymethyl)-aza->-decyl]-pentan
Den freien Komplexbildner erhält man nach der für Beispiel 1o) gegebenen Vorschrift in 72,5%iger Ausbeute als farblose kristalline Verbindung vom Schmelzpunkt 155 ° C.

| Analyse: | | | | |
|---|---|---|---|---|
| Ber.:<br>Gef.: | C 46,54<br>C 46,95 | H 6,34<br>H 6,37 | N 9,67<br>N 9,63 | O 37,44 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 35,91<br>C 35,61 | H 4,44<br>H 4,47 | N 7,46<br>N 7,45 | O 28,89 | Gd 23,27<br>Gd 23,24 |

| Natrium-Salz des Gd-Komplexes | | | | | | |
|---|---|---|---|---|---|---|
| Ber.:<br>Gef.: | C 33,51<br>C 33,51 | H 3,84<br>H 3,80 | N 6,96<br>N 6,94 | O 26,96 | Na 6,98<br>Na 6,91 | Gd 21,72<br>Gd 21,57 |

| N-Methylglucamin-Salz des Gd-Komplexes | | | | | |
|---|---|---|---|---|---|
| Ber.:<br>Gef.: | C 38,69<br>C 38,87 | H 6,12<br>H 6,12 | N 7,35<br>N 7,40 | O 33,59 | Gd 14,23<br>Gd 14,17 |

**BEISPIEL 3**

a) 3-Aza-1,5-diamino-1-(4-hydroxybenzyl)-N,N,N,N,N-pentakis-[2-hydroxy-4-oxa-6,10-bis-<di-(tert.-butoxycarbonylmethyl)-amino>-8-<(tert.-butoxycarbonylmethyl)-aza>-decyl]-pentan
Analog zur Darstellung von Beispiel 2f) wird ausgehend von den Beispielen 1e) und 2e) die Titelverbindung in 78,3%iger Ausbeute als farbloses Öl erhalten.

| Analyse: | | | | |
|---|---|---|---|---|
| Ber.:<br>Gef.: | C 60,21<br>C 60,59 | H 9,15<br>H 9,09 | N 6,28<br>N 6,22 | O 24,34 |

b) 3-Aza-1,5-diamino-1-(4-hydroxybenzyl)-N,N,N,N,N-pentakis-[2-hydroxy-4-oxa-6,10-bis-<di-(carboxymethyl)-amino>-8-<(carboxymethyl)-aza>-decyl]-pentan
Analog zur Darstellung von Beispiel 2g wird die Titelverbindung in 80%iger Ausbeute erhalten. Schmelzpunkt 185 ° C (Zersetzung)

| Analyse: | | | | |
|---|---|---|---|---|
| Ber.:<br>Gef.: | C 46,54<br>C 47,03 | H 6,34<br>H 6,41 | N 9,67<br>N 9,51 | O 37,44 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 35,91<br>C 35,93 | H 4,44<br>H 4,46 | N 7,46<br>N 7,50 | O 28,89 | Gd 23,27<br>Gd 23,11 |

| Natrium-Salz des Gd-Komplexes | | | | | | |
|---|---|---|---|---|---|---|
| Ber.:<br>Gef.: | C 33,51<br>C 33,55 | H 3,84<br>H 3,85 | N 6,96<br>N 6,90 | O 26,96 | Na 6,98<br>Na 7,02 | Gd 21,72<br>Gd 21,66 |

| N-Methylglucamin-Salz des Gd-Komplexes | | | | | |
|---|---|---|---|---|---|
| Ber.:<br>Gef.: | C 38,69<br>C 38,48 | H 6,12<br>H 6,35 | N 7,35<br>N 7,36 | O 33,59 | Gd 14,23<br>Gd 14,26 |

**BEISPIEL 4**

a) 1-(4-Hydroxybenzyl)-N,N,N,N-tetrakis-<2-hydroxy-4-oxo-6,10-bis-[di-(tert.-butoxycarbonylmethyl)-amino]-8-[(tert.-butoxycarbonylmethyl)-aza]-decyl>-1,2-diamino-ethan

5,17 g (21,6 mMol) 1-(4-Hydroxybenzyl)-1,2-diamino-ethan (als Dihydrochlorid) werden in 200 ml Methanol unter Zugabe von 2,4 g (23,8 mMol) Triethylamin gelöst. Bei Raumtemperatur gibt man diese Lösung langsam zu 98,55 g (129,7 mMol) der Verbindung nach Beispiel 2e) in 500 ml Methanol und erwärmt über Nacht am Rückfluß. Danach engt man ein, nimmt in Ether auf und filtriert von unlöslichen Salzen ab. Anschließend reinigt man durch präparative Mitteldruckchromatographie mit Ether/Hexan/Triethylamin. Reste des Amins entfernt man durch eine anschließende Filtration über Kieselgel. Es bleibt ein farbloses Öl zurück (38,8 g, 12,1 mMol, 56% der Theorie).

| Analyse: | | | | |
|---|---|---|---|---|
| Ber.:<br>Gef.: | C 60,31<br>C 60,87 | H 9,11<br>H 9,04 | N 6,11<br>N 6,10 | O 24.45 |

b) 1-(4-Hydroxybenzyl)-N,N,N,N,-tetrakis-<2-hydroxy-4-oxo-6,10-bis -[di-(carboxymethyl)-amino]-8-[-(carboxymethyl)-aza]-decyl>-1,2-diamino-ethan

11,56 g (3,61 mMol) des nach 4a) erhaltenen tert.-Butylesters werden in 100 ml Trifluoressigsäure über Nacht unter Rühren auf 55 °C erwärmt. Man gießt dann in 1 Liter trockenen Ether. Man erhält 8,31 g (2,79 mMol) farblose kristalline Substanz (77,3 % der Theorie).

| Analyse: | | | | |
|---|---|---|---|---|
| Ber.:<br>Gef.: | C 46,68<br>C 46,89 | H 6,28<br>H 6,33 | N 9,40<br>N 9,34 | O 37,61 |

<u>Gadolinium-Komplex</u>

Man löst 4,23 g (1,42 mMol) des Komplexbildners in 100 ml Wasser, das 0,01 Mol Ammonacetat enthält (pH 4,5) auf und versetzt langsam unter Rühren mit 14,23 ml einer 0,1 n Gadoliniumacetatlösung. Nach 15 Minuten stellt man mit Ammoniak den pH-Wert auf 7,5-8 ein, erhitzt 15 Minuten auf 80-90 °C und zentrifugiert. Der Überstand wird gefriergetrocknet und ergibt 5,08 g weiße kristalline Masse (95% der Theorie).

| Analyse: | | | | | |
|---|---|---|---|---|---|
| Ber.: | C 36,02 | H 4,40 | N 7,26 | O 29,02 | Gd 23,28 |
| Gef.: | C 35,73 | H 4,37 | N 7,32 | | Gd 23,20 |

Wie in Beispiel 1o) beschrieben werden erhalten:

| Natrium-Salz des Gd-Komplexes | | | | | | |
|---|---|---|---|---|---|---|
| Ber.: | C 33,56 | H 3,79 | N 6,76 | O 27,04 | Na 7,13 | Gd 21,69 |
| Gef.: | C 33,39 | H 3,75 | N 6,69 | | Na 7,09 | Gd 21,85 |

| N-Methylglucamin-Salz des Gd-Komplexes | | | | | |
|---|---|---|---|---|---|
| Ber.: | C 38,79 | H 6,12 | N 7,22 | O 33,73 | Gd 14,10 |
| Gef.: | C 38,52 | H 6,06 | N 7,29 | | Gd 14,23 |

**BEISPIEL 5**

a) N,N,N,N,N-Pentakis-[5-<2,5,8,11-tetraaza-2,5,8,11-tetrakis-(tert.-butoxycarbonylmethyl)-cyclododecyl>-2-hydroxy-4-oxa-pentyl]-1-[4-(oxiranylmethoxy) benzyl]-3-aza-1,5-diaminopentan
7,56 g (1,99 mMol) der Verbindung nach Beispiel 1n werden in 150 ml Toluol gelöst, mit 60 mg Natriumhydrid (2,0 mMol) 80% in Paraffin und anschließend mit 185 mg (2 mMol) Epichlorhydrin in 20 ml Toluol versetzt. Nach 2 Stunden bei 100 °C kühlt man ab, filtriert die Lösung, wäscht mehrmals mit Wasser und chromatographiert nach Trocknen an Kieselgel mit Ether/Hexan. Man erhält 5,78 g farbloses Öl (75% der Theorie).

| Analyse: | | | | |
|---|---|---|---|---|
| Ber.: | C 60,67 | H 9,26 | N 8,38 | O 21,66 |
| Gef.: | C 60,78 | H 9,30 | N 8,31 | |

b) N,N,N,N,N-Pentakis-[5-<2,5,8,11-tetraaza-2,5,8,11-tetrakis-(carboxymethyl)-cyclododecyl>-2-hydroxy-4-oxa-pentyl]-1-[4-(oxiranyl)-methoxy-benzyl]-3-aza-1,5-diaminopentan
Wie für Beispiel 1o) beschrieben, erhält man den Komplexbildner in 76%iger Ausbeute.

| Analyse: | | | | |
|---|---|---|---|---|
| Ber.: | C 50,37 | H 7,15 | N 11,85 | O 30,61 |
| Gef.: | C 50,64 | H 7,15 | N 11,73 | |

Der Gadolinium-Komplex und dessen Salze werden wie in Beispiel 1o) beschrieben, erhalten.

| Gadolinium-Komplex | | | | | |
|---|---|---|---|---|---|
| Ber.: | C 39,24 | H 5,14 | N 9,23 | O 23,84 | Gd 22,53 |
| Gef.: | C 39,18 | H 5,13 | N 9,28 | | Gd 22,50 |

| Natrium-Salz des Gd-Komplexes | | | | | | |
|---|---|---|---|---|---|---|
| Ber.:<br>Gef.: | C 38,04<br>C 38,14 | H 4,84<br>H 4,80 | N 8,95<br>N 9,00 | O 23,11 | Na 3,19<br>Na 3,20 | Gd 21,84<br>Gd 22,04 |

| N-Methylglucamin-Salz des Gd-Komplexes | | | | | |
|---|---|---|---|---|---|
| Ber.:<br>Gef.: | C 40,08<br>C 40,18 | H 5,93<br>H 5,92 | N 8,78<br>N 8,72 | O 27,59 | Gd 17,60<br>Gd 17,57 |

**BEISPIEL 6**

a) 3-Aza-1,5-diamino-1-[4-(3-methoxycarbonyl)-prop-2-enoxybenzyl]-N,N,N,N,N-pentakis-[2-hydroxy-4-oxa-6,10-bis-<di-(tert.-butoxycarbonylmethyl)-amino>-8-<(tert.-butoxycarbonylmethyl)-aza>-decyl]-pentan
16,32 g (3,72 mMol) der Verbindung nach Beispiel 3a) werden in 250 ml Toluol gelöst, mit 112 mg NaH 80% in Paraffin (3,73 mMol) versetzt und tropfenweise bei 40 - 50 °C mit einer Lösung von 740 mg 4-Bromcrotonsäuremethylester (cis/trans-Isomerengemisch, 90%ig) (3,72 mMol) in 20 ml Toluol versetzt. Nach 2 Stunden Rühren filtriert man, wäscht mit Wasser und chromatographiert an Kieselgel mit Essigester/Hexan. Man erhält 10,98 g (66% der Theorie) als farbloses Öl.

| Analyse: | | | | |
|---|---|---|---|---|
| Ber.:<br>Gef.: | C 60,24<br>C 59,96 | H 9,07<br>H 8,99 | N 6,13<br>N 6,08 | O 24,54 |

b) 3-Aza-1,5-diamino-1-[4-(3-methoxycarbonyl)-prop-2-enoxybenzyl]-N,N,N,N,N-pentakis-[2-hydroxy-4-oxa-6,10-bis-<di-(carboxymethyl)-amino>-8-<(carboxymethyl)-aza>-decyl]-pentan
Wie für Beispiel 1o) beschrieben, erhält man den freien Komplexbildner in 76%iger Ausbeute als weißes kristallines Pulver vom Schmelzpunkt 140 - 143 °C

| Analyse: | | | | |
|---|---|---|---|---|
| Ber.:<br>Gef.: | C 47,07<br>C 46,74 | H 6,33<br>H 6,38 | N 9,32<br>N 9,30 | O 37,26 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 36,63<br>C 36,67 | H 4,49<br>H 4,49 | N 7,25<br>N 7,18 | O 29,00 | Gd 22,62<br>Gd 22,82 |

| Natrium-Salz des Gd-Komplexes | | | | | | |
|---|---|---|---|---|---|---|
| Ber.:<br>Gef.: | C 34,45<br>C 34,20 | H 3,95<br>H 3,98 | N 6,82<br>N 6,88 | O 27,27 | Na 6,22<br>Na 6,21 | Gd 21,27<br>Gd 21,23 |

| N-Methylglucamin-Salz des Gd-Komplexes | | | | | |
|---|---|---|---|---|---|
| Ber.:<br>Gef.: | C 38,94<br>C 38,66 | H 6,03<br>H 6,03 | N 7,22<br>N 7,27 | O 33,30 | Gd 14,48<br>Gd 14,45 |

**BEISPIEL 7**

a) 3-Aza-1-(4-hydroxybenzyl)-N,N,N-tri-tosyl-pentan-1,5-diamin
24,0 g (75,3 mMol) 3-Aza-1-(4-hydroxybenzyl)-pentan-1,5-diamin . Trihydrochlorid (Beispiel 1e) werden in 250 ml trockenem Pyridin vorgelegt und bei 0°C unter gutem Rühren 37,9 g (198,7 mMol) Tosylchlorid, gelöst in 100 ml Pyridin, über eine Stunde zugetropft. Man läßt über Nacht bei 4°C stehen, dampft den Hauptteil des Pyridins im Vakuum ab und nimmt in 300 ml Dichlormethan auf. Durch mehrmaliges Waschen mit verdünnter Salzsäure, wäßriger Bicarbonatlösung und bidestilliertem Wasser entfernt man restliches Pyridin und überschüssige Toluolsulfonsäure. Nach Trocknen wird an Kieselgel mit Essigester/Hexan chromatographiert. Man erhält das Tritosylat in Form farbloser Kristalle. Ausbeute 36,9 g (73% der Theorie)
Schmelzpunkt: 145-146°C

| Analyse: | | | | | |
|---|---|---|---|---|---|
| Ber.:<br>Gef.: | C 57,20<br>C 57,15 | H 5,55<br>H 5,32 | N 6,25<br>N 6,24 | O 16,66 | S 14,31<br>S 14,20 |

b) 2-(4-Hydroxybenzyl)-1,4,7,10-tetra-tosyl-1,4,7,10-tetraazacyclododecan
10,95 g (16,3 mMol) 3-Aza-1-(4-hydroxybenzyl)-N,N,N-tri-tosyl-pentan-1,5-diamin werden in 100 ml Dimethylformamid gelöst und mit 1,35 g (45 mMol) Natriumhydrid (80% in Paraffin) 30 Minuten bei 35-40°C gerührt. Danach versetzt man langsam mit einer Lösung von 9,51 g (16,3 mMol) 3-Aza-1,3,5-tri-tosyl-1,5-dihydroxypentan in 100 ml DMF. Man rührt vier Stunden bei 130°C, läßt über Nacht erkalten und destilliert das Lösungsmittel ab. Der Rückstand kristallisiert beim Verreiben mit wenig Methanol. Nach Waschen mit verdünnter Salzsäure und Umkristallisieren aus Acetonitril erhält man 7,4 g (48% der Theorie) farblose Kristalle vom Schmelzpunkt 192-193°C.

| Analyse: | | | | | |
|---|---|---|---|---|---|
| Ber.:<br>Gef.: | C 53,84<br>C 53,66 | H 5,25<br>H 5,17 | N 5,84<br>N 5,81 | O 21,68 | S 13,37<br>S 13,30 |

c) 2-(4-Hydroxybenzyl)-1,4,7,10-tetraazacyclododecan . Trihydrobromid
12,0 g (12,7 mMol) 2-(4-Hydroxybenzyl-1,4,7,10-tetra-tosyl-1,4,7,10-tetraazacyclododecan werden in 50 ml Eisessig mit 33% Bromwasserstoff vier Stunden auf 100°C erhitzt. Man gießt in 300 ml Diethylether, saugt ab und resuspendiert zweimal in jeweils 300 ml Diethylether. Alle Operationen werden unter Stickstoffschutzatmosphäre durchgeführt. Nach dem Trocknen im Vakuum liegen 5,16 g (78% der Theorie) farbloser Kristalle vor, die bei 115-117°C unter Zersetzung schmelzen.

| Analyse: | | | | | |
|---|---|---|---|---|---|
| Ber.:<br>Gef.: | C 34,57<br>C 34,75 | H 5,60<br>H 5,61 | N 10,75<br>N 10,77 | O 3,07 | Br 45,99<br>Br 45,64 |

d) 3,6-Diaza-3,6-bis-(tert.-butoxycarbonylmethyl)-4-(4-hydroxybenzyl)-suberinsäure-bis-(tert.-butyl)-diester
15,31 g (0,064 Mol) 4-Hydroxybenzyl-1,2-ethan-diamin als Dihydrochlorid und 71,14 g (0,71 Mol) Kaliumhydrogencarbonat werden in 380 ml Dimethylformamid (getrocknet über Natriumhydrid) vorgelegt und bei 35°C 50 g (0,26 Mol) Bromessigsäure-tert.-butylester in 80 ml Dimethylformamid zugetropft. Man rührt noch weitere 2,5 Stunden bei 35°C, wonach kein Ausgangsprodukt mehr dünnschichtchromatographisch nachzuweisen ist. Man filtriert von ausgefallenem Kaliumbromid ab und engt das Filtrat ein.

Der Rückstand wird mit Wasser versetzt und mehrmals mit Ether extrahiert. Nach Trocknen und Einengen wird der Etherextrakt über eine Kieselgelsäule von unumgesetzten Bromessigsäure-tert.-butylester gereinigt. Man erhält 24,8 g (63% der Theorie) eines farblosen Öls.

| Analyse: | | | | |
|---|---|---|---|---|
| Ber.:<br>Gef.: | C 63,64<br>C 63,78 | H 8,73<br>H 8,69 | N 4,49<br>N 4,41 | O 23,12 |

e) 3,6-Diaza-3,6-bis-(tert.-butoxycarbonylmethyl)-4-(4-oxiranylmethoxy)-benzyl-suberinsäure-bis-(tert.-butyl)-diester

17,38 g (27,9 mMol) 3,6-Diaza-3,6-bis-(tert.-butoxycarbonylmethyl)-4-(4-hydroxybenzyl)-suberinsäure-bis-(tert.-butyl)-diester (Beispiel 7d) werden mit 900 mg (30 mMol) 80% Natriumhydrid in Paraffin in 300 ml Toluol unter Rühren gelöst und bei 40 °C tropfenweise mit einer Lösung von 2,6 g (28 mMol) Epichlorhydrin versetzt. Nach einer Stunde wird vorsichtig mit 100 ml Wasser versetzt. Nach Schütteln werden die Phasen getrennt und anschließend wird die organische Phase nach Trocknen eingeengt. Nach chromatographischer Reinigung liegen 15,5 g (22,8 mMol, 82% der Theorie) als farbloses Öl vor.

| Analyse: | | | | |
|---|---|---|---|---|
| Ber.:<br>Gef.: | C 63,69<br>C 63,57 | H 8,61<br>H 8,62 | N 4,12<br>N 4,07 | O 23,56 |

f) 2-Hydroxybenzyl-1,4,7,10-tetraaza-N,N,N,N-tetrakis-2-hydroxy-3-{4-(2,3-bis-<bis-(tert.-butoxycarbonylmethyl)-amino>-propyl}-phenoxypropyl -cyclododecan

Analog der Vorschrift für Beispiel 1n) werden 17,35 mMol 2-(4-Hydroxybenzyl)-1,4,7,10-tetraazacyclododecan. Hydrobromid (Beispiel 7c) in Methanol/THF mit 4 Equivalenten der Verbindung nach Beispiel 7e) in Anwesenheit von Triethylamin als Säurebinder umgesetzt. Die Ausbeute beträgt 63% der Theorie; es handelt sich um ein hochviskoses, schwachgelbes Öl.

| Analyse: | | | | |
|---|---|---|---|---|
| Ber.:<br>Gef.: | C 63,45<br>C 63,91 | H 8,45<br>H 8,41 | N 5,72<br>N 5,74 | O 22,35 |

g) 2-Hydroxybenzyl-1,4,7,10-tetraaza-N,N,N,N-tetrakis-2-hydroxy-3-{4-(2,3-bis-<bis-(carboxymethyl)-amino>-propyl}-phenoxypropyl -cyclododecan

Analog der Vorschrift für Beispiel 1o) erhält man in 71%iger Ausbeute den freien Komplexbildner durch Behandeln mit warmer Trifluoressigsäure als weißes, feinkristallines Pulver vom Schmelzpunkt 187-189 °C.

| Analyse: | | | | |
|---|---|---|---|---|
| Ber.:<br>Gef.: | C 53,68<br>C 54,03 | H 5,84<br>H 5,79 | N 8,25<br>N 8,19 | O 32,21 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 41,20<br>C 41,22 | H 4,02<br>H 4,00 | N 6,33<br>N 6,33 | O 24,72 | Gd 23,70<br>Gd 23,59 |

| Natrium-Salz des Gd-Komplexes | | | | | | |
|---|---|---|---|---|---|---|
| Ber.:<br>Gef.: | C 39,56<br>C 39,39 | H 3,68<br>H 3,71 | N 6,08<br>N 6,07 | O 23,74 | Na 4,16<br>Na 4,12 | Gd 22,76<br>Gd 22,63 |

| N-Methylglucamin-Salz des Gd-Komplexes | | | | | |
|---|---|---|---|---|---|
| Ber.:<br>Gef.: | C 41,70<br>C 41,74 | H 5,30<br>H 5,33 | N 6,56<br>N 6,52 | O 29,09 | Gd 17,33<br>Gd 17,43 |

**BEISPIEL 8**

a) 3-Aza-1-hydroxymethyl-1,3,5-tris-(trifluoro-acetyl)-pentan-1,5-diamin
43,7 g (137,13 mMol) der Verbindung nach Beispiel 1h) werden in 500 ml Ethanol suspendiert, mit 48,5 g (480 mMol) Triethylamin und 77,74 g (547,15mMol) Trifluoressigsäureethylester 7 Tage am Rückfluß gekocht. Danach ersetzt man das Lösungsmittel durch Essigester, wäscht mit verdünnter Salzsäure und Wasser, entfärbt mit Aktivkohle, trocknet und engt ein. Es hinterbleiben 44,43 g (105,73 mMol) farblose Kristalle (77% der Theorie).
Schmelzpunkt: 179-180 ° C

| Analyse: | | | | | |
|---|---|---|---|---|---|
| Ber.:<br>Gef.: | C 31,36<br>C 31,32 | H 2,87<br>H 2,91 | N 9,98<br>N 10,02 | O 15,19 | F 40,59<br>F 40,42 |

b) 3-Aza-1-(oxiranylmethoxy)-methyl-1,3,5-tris-(trifluoroacetyl)-pentan-1,5-diamin
32,17 g (76,56 mMol) der Titelverbindung von 8a) werden in 480 ml Tetrahydrofuran mit 3,29 g (84,2 mMol) Natriumamid bei Raumtemperatur 30 Minuten gerührt und dann mit 7,79 g (84,2 mMol) Epichlorhydrin, gelöst in 200 ml Tetrahydrofuran, tropfenweise versetzt. Dannach erwärmt man noch 2 Stunden auf 60 ° C. Nach dem Abziehen des Lösungsmittels nimmt man in Essigester auf, filtriert Unlösliches ab und reinigt durch Säulenchromatographie. Man erhält 25,39 g (53,2 mMol) farblose Kristalle (69,5 % der Theorie)
Schmelzpunkt: 85-87 ° C

| Analyse: | | | | | |
|---|---|---|---|---|---|
| Ber.:<br>Gef.: | C 35,23<br>C 35,54 | H 3,37<br>H 3,36 | N 8,80<br>N 8,74 | O 16,76 | F 35,82<br>F 35,76 |

c) 3-Aza-1-(4-hydroxybenzyl)-N,N,N,N,N-pentakis-<8-aza-2-hydroxy-4-oxa-N,N,N-tris-(trifluoracetyl)-6,10-diamino-decyl>-pentan-1,5-diamin
6,35 g (19,93 mMol) der Verbindung nach Beispiel 1e) werden mit 6,64 g (65,76 mMol) Triethylamin in 150 ml trockenem Tetrahydrofuran gelöst und langsam zu einer siedenden Lösung von 58,97 g (123,54 mMol) der nach 8b) erhaltenen Verbindung in 600 ml Methanol gegeben. Nach 48 Stunden Rückfluß wird abgekühlt, das Lösungsmittel eingeengt und der Rückstand mit Essigester extrahiert. Nach Säulenchromatographie erhält man 37,4 g (14,41 mMol) farbloser Festsubstanz (72,3% der Theorie).
Schmelzpunkt. 75-80 ° C

| Analyse: | | | | | |
|---|---|---|---|---|---|
| Ber.:<br>Gef.: | C 37,48<br>C 37,53 | H 3,84<br>H 3,87 | N 9,71<br>N 9,80 | O 16,02 | F 32,93<br>F 32,63 |

d) 3-Aza-1-(4-hydroxybenzyl)-N,N,N,N,N-pentakis-<8-aza-2-hydroxy-4-oxa-6,10-diamino-decyl>-pentan-1,5-diamin

30,61 g (11,79 mMol) der in der vorigen Reaktionsstufe erhaltenen Verbindung werden in 500 ml Ethanol suspendiert und portionsweise mit insgesamt 26,77 g (707,5 mMol) Natriumborhydrid versetzt. Man rührt 2 Tage bei 35-40 ° C, versetzt dann mit 200 ml Aceton, dampft das Lösungsmittel ab und versetzt mit 500 ml 1 n Natronlauge. Fünfmalige Extraktion mit insgesamt 2 Liter Essigester liefert nach Trocknen und Einengen der organischen Phase einen öligen Rückstand. Man erhält 9,17 g (7,94 mMol) Substanz (67,5% der Theorie).

| Analyse: | | | | |
|---|---|---|---|---|
| Ber.:<br>Gef.: | C 53,00<br>C 52,80 | H 9,94<br>H 9,90 | N 21,81<br>N 21,68 | O 15,22 |

e) 3-Aza--1,5-diamino-2-(4-hydroxybenzyl)-N,N,N,N,N-pentakis-<8-aza-6,10-diamino-2-hydroxy-4-oxa-N′,N′,N′,N′,N′-pentakis- 2-hydroxy-4-oxa-6,10-bis-{di(carboxymethyl)-amino}-8-{carboxymethyl)-aza}-decyl -decyl>-pentan

8,36 g (7,23 mMol) der in der vorigen Reaktionsstufe erhaltenen Verbindung werden mit 800 mg (7,96 mMol) Triethylamin in 85 ml Methanol gelöst und zu 192,4 g (253,2 mMol) 3,6,9-Triaza-3,6,9-tris-(tert.-butoxycarbonylmethyl)-4-(oxiranylmethoxy)-methyl-undecandisäure-bis-(tert.-butyl)-diester (Beispiel 2e) gelöst in 2000 ml Methanol, zugegeben. Man erhitzt 36 Stunden am Rückfluß und entfernt das Lösungsmittel am Rotationsverdampfer. Danach löst man das zurückbleibende Öl unter leichtem Erwärmen in 500 ml Trifluoressigsäure und hält über Nacht bei 60 ° C. Man gießt unter Rühren in 3 l trockenen Diethylether, saugt den Niederschlag ab und nimmt ihn in 200 ml Wasser auf. Nach Neutralisieren mit Ammoniaklösung unterwirft man die Lösung einer Dialyse. Das Retentat ergibt nach Gefriertrocknung 56,75 g (43,2 % der Theorie) weißer feinkristalliner Masse,die oberhalb 110 ° C unter Braunfärbung sintert. Das titrimetrisch mit Xylenolorange ermittelte Komplexierungsvermögen für Gadolinium bei pH 5 beträgt 223,7 mg Gd/g.

| Analyse: | | | | |
|---|---|---|---|---|
| Ber.:<br>Gef.: | C 45,09<br>C 45,10 | H 7,84<br>H 7,81 | N 9,76<br>N 9,73 | O 37,29 |

Gadolinium-Komplex

Man löst 6,27 g (0,35 mMol) des vorstehend beschriebenen Komplexbildners in 100 ml 0,1 m Natriumacetatlösung pH 5 auf und versetzt mit 8,8 ml einer 1,037 n Lösung von Gadoliniumacetat in Wasser. Nach Dialyse wird gefriergetrocknet. Es bleiben 7,7 g weißes Pulver zurück, das einen Gadoliniumgehalt von 179,6 mg/g aufweist.

| Analyse: | | | | | |
|---|---|---|---|---|---|
| Ber.:<br>Gef.: | C 34,98<br>C 34,86 | H 5,64<br>H 5,65 | N 7,57<br>N 7,51 | O 28,93 | Gd 22,85<br>Gd 22,71 |

Na-Salz des Gd-Komplexes

Eine Lösung des vorstehend beschriebenen Gadolinium-Komplexes (3,55 g in 17 ml Wasser) werden mit 0,1 n Natronlauge auf pH 7,0-7,1 eingestellt und gefriergetrocknet. Man erhält 3,71 g (99,4 % der Theorie) eines voluminösen weißen Pulvers.

| Analyse: | | | | | | |
|---|---|---|---|---|---|---|
| Ber.:<br>Gef.: | C 32,84<br>C 33,02 | H 5,02<br>H 5,01 | N 7,11<br>N 7,13 | O 27,16 | Na 6,40<br>Na 6,34 | Gd 21,45<br>Gd 21,62 |

Meglumin-Salz des Gd-Komplexes

Wie für das Na-Salz beschrieben, erhält man auch das Meglumin-Salz des Komplexes durch Neutralisieren. Für 12,61 g Komplex werden 5,7 g (29,07 mMol) N-Methylglucamin verbraucht. Nach Dialyse und Gefriertrocknen liegen 18,29 weiße feinkristalline Substanz vor.

| Analyse: | | | | | |
|---|---|---|---|---|---|
| Ber.:<br>Gef.: | C 37,95<br>C 37,71 | H 6,79<br>H 6,83 | N 7,42<br>N 7,39 | O 33,34 | Gd 14,47<br>Gd 14,53 |

**BEISPIEL 9**

a) N,N,N,N-Tetrakis-<5-[2,5,8,11-tetrakis-(tert.-butoxycarbonylmethyl)-2,5,8,11 tetraaza-cyclododecyl]-2-hydroxy-4-oxa-pentamethylen>-1,2-diaminoethan
83 mg (1,38 mMol) Ethylendiamin werden in 50 ml Tetrahydrofuran vorgelegt und unter Kühlung mit 4,0 g (5,58 mMol) der Verbindung von Beispiel 1m) in 30 ml Tetrahydrofuran bei Eiskühlung tropfenweise versetzt. Man läßt über Nacht auf Raumtemperatur erwärmen, entfernt das Lösungsmittel und reinigt über eine Kieselgelsäule von unumgesetztem Ausgangsmaterial. Man erhält 288 mg (71% der Theorie) analysenreines Material als farbloses Öl.

| Analyse: | | | | |
|---|---|---|---|---|
| Ber.:<br>Gef.: | C 60,05<br>C 59,83 | H 9,39<br>H 9,29 | N 8,64<br>N 8,70 | O 21.92 |

b) N,N,N,N-Tetrakis-<5-[2,5,8,11-tetrakis-(carboxymethyl)-2,5,8,11-tetraaza-cyclododecyl]-2-hydroxy-4-oxa-pentamethylen>-1,2-diaminoethan
Wie für Beispiel 1o) beschrieben, gelingt die Abspaltung der Ester mit Trifluoressigsäure. Nach Umkristallisation erhält man in 82% der Theorie die freie Säure als weißes Pulver vom Schmelzpunkt 183 °C.

| Analyse: | | | | |
|---|---|---|---|---|
| Ber.:<br>Gef.: | C 48,70<br>C 48,79 | H 7,17<br>H 7,20 | N 12,46<br>N 12,46 | O 31,64 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 37,32<br>C 37,25 | H 5,04<br>H 5,03 | N 9,55<br>N 9,60 | O 24,25 | Gd 23,83<br>Gd 23,79 |

| Natrium-Salz des Gd-Komplexes | | | | | | |
|---|---|---|---|---|---|---|
| Ber.:<br>Gef.: | C 36,11<br>C 35,87 | H 4,73<br>H 4,72 | N 9,24<br>N 9,25 | O 23,46 | Na 3,37<br>Na 3,33 | Gd 23,06<br>Gd 23,05 |

| N-Methylglucamin-Salz des Gd-Komplexes | | | | | |
|---|---|---|---|---|---|
| Ber.:<br>Gef.: | C 38,63<br>C 38,27 | H 5,89<br>H 5,90 | N 9,01<br>N 9,06 | O 28,06 | Gd 18,39<br>Gd 18,40 |

**BEISPIEL 10**

a) 3,6,9-Triaza-4-(4-hydroxybenzyl)-3,6,9-tris-(tert.-butoxycarbonylmethyl)-undecandisäure-bis-(tert.-butyl)-diester

2,07 g (6,5 mMol) 3-Aza-1-(4-hydroxybenzyl)-pentan-1,5-diamin . Trihydrochlorid (Beispiel 1e) werden mit 5,2 g Natriumhydrogencarbonat und 6,34 g (82,2 mMol) Bromessigsäure-tert.-butylester nach der Vorschrift für Beispiel 2d) hergestellt.

Man erhält 3,54 g (68,8% der Theorie) eines farblosen Öls.

| Analyse: | | | | |
|---|---|---|---|---|
| Ber.:<br>Gef.: | C 63,13<br>C 63,21 | H 8,91<br>H 8,90 | N 5,38<br>N 5,42 | O 22,56 |

b) 3,6,9-Triaza-3,6,9-tris-(tert.-butoxycarbonylmethyl)-4-(4-oxiranylmethoxybenzyl)-undecandisäure-bis-(tert.-butyl)-diester

16,35 g (21,0 mMol) 3,6,9-Triaza-3,6,9-tris-(tert.-butoxycarbonylmethyl)-4-(4-hydroxybenzyl)-undecandisäure-bis-(tert.-butyl)-diester (Beispiel 10a) werden mit 630 mg (21 mMol) Natriumhydrid (80% in Paraffin) in 300 ml Toluol unter Rühren gelöst und bei 40 ° C tropfenweise mit einer Lösung von 1,95 g (21 mMol) Epichlorhydrin in 20 ml Toluol versetzt. Nach Aufarbeitung wie für Beispiel 5 a beschrieben, liegen 15,4 g (88% der Theorie) farbloses Öl vor.

| Analyse: | | | | |
|---|---|---|---|---|
| Ber.:<br>Gef.: | C 63,20<br>C 63,35 | H 8,80<br>H 8,76 | N 5,02<br>N 5,09 | O 22,96 |

c) N,N,N,N-Tetrakis-{2-hydroxy-3-<4-{2,6-bis-(di-(tert.-butoxycarbonylmethyl)-amino)-4-((tert.-butoxycarbonylmethyl)-aza)-hexyl}-phenoxy>-trimethylen}-1,2 diaminoethan

720 mg (12,0 mMol) Ethylendiamin werden in 125 ml Tetrahydrofuran vorgelegt und unter Eiskühlung mit 40,13 g (48 mMol) der nach 10b) erhaltenen Verbindung versetzt. Man läßt über Nacht aufwärmen, zieht das Lösungsmittel ab und chromatographiert an Kieselgel. Man erhält 21,65 g (53% der Theorie) eines farblosen Öls.

| Analyse: | | | | |
|---|---|---|---|---|
| Ber.:<br>Gef.: | C 62,79<br>C 63,98 | H 8,88<br>H 8,75 | N 5,76<br>N 5,70 | O 22,55 |

d) N,N,N,N-Tetrakis-{2-hydroxy-3-<4-{2,6-bis-(di-(carboxymethyl)-amino)-4-((carboxymethyl)-aza)-hexyl}-phenoxy>-trimethylen}-1,2-diaminoethan

Nach der für Beispiel 1o) gegebenen Vorschrift erhält man die Titelverbindung in 68%iger Ausbeute als weißes, kristallines Pulver vom Schmelzpunkt 180 ° C (Zersetzung).

| Analyse: | | | | |
|---|---|---|---|---|
| Ber.:<br>Gef.: | C 51,57<br>C 51,25 | H 6,18<br>H 6,12 | N 8,59<br>N 8,58 | O 33,64 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 40,60<br>C 40,45 | H 4,45<br>H 4,44 | N 6,76<br>N 6,70 | O 26,48 | Gd 21,69<br>Gd 21,71 |

| Natrium-Salz des Gd-Komplexes | | | | | | |
|---|---|---|---|---|---|---|
| Ber.:<br>Gef.: | C 38,27<br>C 38,04 | H 3,93<br>H 3,96 | N 6,37<br>N 6,37 | O 24,97 | Na 5,98<br>Na 5,93 | Gd 20,45<br>Gd 20,34 |

| N-Methylglucamin-Salz des Gd-Komplexes | | | | | |
|---|---|---|---|---|---|
| Ber.:<br>Gef.: | C 41,46<br>C 41,05 | H 5,96<br>H 5,94 | N 6,90<br>N 6,94 | O 31,56 | Gd 14,10<br>Gd 14,09 |

**BEISPIEL 11**

a) 3-Aza-1,5-diamino-2-{4-(3-benzyloxycarbonylaminopropoxy)-benzyl}-N,N,N,N,N-pentakis-{2-hydroxy-4-oxa-6,10-bis-<di-(tert.-butoxycarbonylmethyl)-amino>-8-< (tert.-butoxycarbonylmethyl)-aza>-decyl}-pentan

21,7 g (5,41 mMol) dere Verbindung nach Beispiel 2f) werden mit 170 mg (5,7 mMol) Natriumhydrid (80% in Paraffinöl) in 250 ml Toluol gelöst und langsam bei 30 ° C mit einer Lösung von 1,62 g (5,95 mMol) N-(3-Brompropyl)-carbaminsäurebenzylester in 30 ml Toluol versetzt. Nach 2 Stunden wird eingeengt und über einer Kieselgelsäule gereinigt. Man erhält 16,49 g (3,92 mMol) eines farblosen Öls (72,5% der Theorie).

| Analyse: | | | | |
|---|---|---|---|---|
| Ber.:<br>Gef.: | C 60,60<br>C 60,29 | H 9,06<br>H 9,00 | N 6,33<br>N 6,39 | O 23,99 |

b) 3-Aza-1,5-diamino-2-{4-(3-aminopropoxy)-benzyl}-N,N,N,N,N-pentakis-{2-hydroxy-4-oxa-6,10-bis-<di-(tert.-butoxycarbonylmethyl)-amino>-8<(tert.-butoxycarbonylmethyl)-aza>-decyl}-pentan

12,55 g (2,99 mMol) der nach 11a) erhaltenen Verbindung werden in 180 ml Methanol mit 1 g 10% Palladium-Kohle hydriert, bis keine weitere Wasserstoffaufnahme mehr erfolgt. Nach Abfiltrieren des Katalysators und Einengen bleiben 11,38 g (2,80 mMol) 93,7% der Theorie) als farbloses Öl zurück.

| Analyse: | | | | |
|---|---|---|---|---|
| Ber.:<br>Gef.: | C 60,24<br>C 59,67 | H 9,21<br>H 9,24 | N 6,54<br>N 6,53 | O 23,99 |

c) 3-Aza-1,5-diamino-2-{4-(3-maleimidopropoxy)-benzyl}-N,N,N,N,N-pentakis-{2-hydroxy-4-oxa-6,10-bis-<di-(tert.-butoxycarbonylmethyl)-amino>-8-<(tert.-butoxycarbonylmethyl)-aza>-decyl}-pentan

9,8 g (2,41 mMol) der Titelverbindung von 11b) in 200 ml Dichlormethan werden mit 261 mg (2,65 mMol)

Maleinsäureanhydrid über Nacht bei 40 ° C gerührt und anschließend bei Raumtemperatur mit 330 mg (2,41 mMol) N-Hydroxybenzotriazol und 500 mg (2,41 mMol) Dicyclohexylcarbodiimid versetzt. Nach 2 Tagen filtriert man und reinigt säulenchromatographisch. Man erhält 7,59 g(1,83 mMol) farbloses Öl (76% der Theorie).

| Analyse: | | | | |
|---|---|---|---|---|
| Ber.:<br>Gef.: | C 60,23<br>C 59,88 | H 9,04<br>H 9,12 | N 6,41<br>N 6,45 | O 24,3 |

3-Aza-1,5-diamino-2-{4-(3-maleimidopropoxy)-benzyl}-N,N,N,N,N-pentakis-{2-hydroxy-4-oxa-6,10-bis-<di-(carboxymethyl)-amino>-8-<(carboxymethyl)-aza>-decyl}-pentan

8,23 g (1,98 mMol) der nach 11c) erhaltenen Verbindung werden mit 100 ml Trifluoressigsäure 25 Stunden bei 40 C gerührt. Danach gießt man in 1,5 Liter trockenen Diethylether, saugt ab und wäscht auf der Nutsche mit Ether säurefrei. Nach Trocknen im Vakuum liegen 5,14 g (1,87 mMol) weißer feinkristalliner Substanz vor (94,4% der Theorie).
Schmelzpunkt: 212-214 ° C (Zersetzung)

| Analyse: | | | | |
|---|---|---|---|---|
| Ber.:<br>Gef.: | C 47,27<br>C 47,69 | H 6,28<br>H 6,26 | N 9,69<br>N 9,75 | O 36,73 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 36,90<br>C 37,01 | H 4,47<br>H 4,44 | N 7,57<br>N 7,49 | O 28,67 | Gd 22,36<br>Gd 22,56 |

| Natrium-Salz des Gd-Komplexes | | | | | | |
|---|---|---|---|---|---|---|
| Ber.:<br>Gef.: | C 34,73<br>C 34,45 | H 3,94<br>H 3,93 | N 7,12<br>N 7,09 | O 26,98 | Na 6,15<br>Na 6,16 | Gd 21,05<br>Gd 21,12 |

| N-Methylglucamin-Salz des Gd-Komplexes | | | | | |
|---|---|---|---|---|---|
| Ber.:<br>Gef.: | C 39,10<br>C 38,82 | H 6,01<br>H 6,03 | N 7,43<br>N 7,35 | O 33,07 | Gd 14,38<br>Gd 14,25 |

**BEISPIEL 12**

a) 1-(4-Benzyloxycarbonylmethoxybenzyl)-N,N,N,N-tetrakis-<2-hydroxy-4-oxa-6,10-bis-{di-(tert.-butoxycarbonylmethyl)-amino}-8-{(tert.-butoxycarbonylmethyl)-aza}-decyl>-1,2-diamino-ethan

13,85 g (4,31 mMol) der Verbindung nach Beispiel 4a) werden in 150 ml Toluol gelöst und mit 168,1 mg (4,31 mMol) gepulvertem Natriumamid vorsichtig ver setzt. Danach tropft man eine Lösung von 1,04 g (4,54 mMol) Bromessigsäurebenzylester in 10 ml Toluol zu und erwärmt 2 Stunden auf 80 ° C. Danach saugt man ab, wäscht mehrmals mit Wasser und engt nach Trocknen ein. Es hinterbleiben 13,1 g eines gelben Öls, das durch Chromatographie an Kieselgel weiter gereinigt wird. Man erhält schließlich 11,4 g (3,41 mMol) farbloses, viskoses Öl (78,7% der Theorie).

| Analyse: | | | | |
|---|---|---|---|---|
| Ber.:<br>Gef.: | C 60,87<br>C 61,12 | H 8,95<br>H 9,03 | N 5,84<br>N 5,79 | O 24,32 |

b) 1-(4-Hydrazinocarbonylmethoxybenzyl)-N,N,N,N-tetrakis-<2-hydroxy-4-oxa-6,10-bis-{di-(tert.-butoxycarbonylmethyl)-amino}-8-{(tert.-butoxycarbonylmethyl)-aza}-decyl>-1,2-diamino-ethan
19,24 g (5,74 mMol) der nach 12a) erhaltenen Verbindung werden in 250 ml absolutem Ethanol gelöst und mit einer Lösung von 573 mg (11,47 mMol) Hydrazin-Monohydrat in 5 ml Ethanol bei max. 10°C versetzt. Nach 18 Stunden bei Raumtemperatur engt man auf das halbe Volumen ein und gießt in 2 Liter Wasser. Das Produkt wird mit Ether extrahiert und über eine Kieselgelsäule gereinigt. Es werden 16,73 g (5,1 mMol) (89% der Theorie) farbloses Öl erhalten.

| Analyse: | | | | |
|---|---|---|---|---|
| Ber.:<br>Gef.: | C 59,72<br>C 59,15 | H 9,03<br>H 9,08 | N 6,83<br>N 6,80 | O 24,4 |

c) 1-(4-Hydrazinocarbonylmethoxybenzyl)-N,N,N,N-tetrakis-<2-hydroxy-4-oxa-6,10 bis-{di-(carboxymethyl)-amino}-8-{(carboxymethyl)-aza}-decyl>1,2-diaminoethan
11,26 g (3,43 mMol) der nach 12b) erhaltenen Verbindung werden 16 Stunden bei 60°C in 200 ml Trifluoressigsäure gerührt. Man engt auf etwa ein Drittel des Ausgangsvolumens ein und gießt in 1 Liter trockenen Diethylether. Das abgesaugte Produkt wird mit weiterem Ether säurefrei gewaschen und dann getrocknet. Man erhält 6,94 g (3,22 mMol) (93,7% der Theorie) weiße Kristalle.
Schmelzpunkt: 167-169°C (Zersetzung)

| Analyse: | | | | |
|---|---|---|---|---|
| Ber.:<br>Gef.: | C 46,23<br>C 46,27 | H 6,26<br>H 6,20 | N 10,39<br>N 10,42 | O 37,1 |

Der Gadolinium-Komplex und dessen Salze werden wie in Beispiel 1o) beschrieben, erhalten.

| Gadolinium-Komplex | | | | | |
|---|---|---|---|---|---|
| Ber.:<br>Gef.: | C 35,95<br>C 35,59 | H 4,43<br>H 4,46 | N 8,08<br>N 8,02 | O 28,84 | Gd 22,68<br>Gd 22,89 |

| Natrium-Salz des Gd-Komplexes | | | | | | |
|---|---|---|---|---|---|---|
| Ber.:<br>Gef.: | C 33,80<br>C 33,52 | H 3,89<br>H 3,87 | N 7,59<br>N 7,59 | O 27,12 | Na 6,23<br>Na 6,28 | Gd 21,33<br>Gd 21,19 |

| N-Methylglucamin-Salz des Gd-Komplexes | | | | | |
|---|---|---|---|---|---|
| Ber.:<br>Gef.: | C 38,51<br>C 38,75 | H 5,99<br>H 5,99 | N 7,75<br>N 7,81 | O 33,21 | Gd 14,51<br>Gd 14,26 |

**BEISPIEL 13**

Konjugat des 3-Aza-1,5-diamino-2-[4-(3-maleimidopropoxy)-benzyl-N,N,N,N-pentakis-[2-hydroxy-4-oxa-6,10-bis-<di-(carboxymethyl)-amino]-8-[carboxymethyl)-aza> decyl]-pentans mit Fab-Fragmenten des monoklonalen Antikörpers 7B10D11

a) Herstellung von $F(ab')_2$-Fragmenten:
16 mg (100nMol) des Antikörpers 7B10D11 werden in 1 ml eines Gemisches von 0,1 m-Acetatpuffer (pH 4,5) und 0,1 M Kochsalzlösung gelöst und nach Zugabe von 0,3 mg Pepsin 20 Stunden bei 37 °C inkubiert. Nach Reinigung über Ultragel ACA (Firma LKB) bei pH 7,0 und nach Gefriertrocknung erhält man 6,3 mg (63% der Theorie) der gewünschten Fragmente).
b) Herstellung und Kopplung von Fab-Fragmenten:
15 mg (150 nMol) der nach a) erhaltenen Fragmente werden in 14,5 ml 0,1 M-Phosphatpuffer (pH 6,0) gelöst und mit 0,15 ml einer 0,1 M-Mercaptoethylaminlösung in 0,1 M-Phosphatpuffer (pH 6,0) unter Zusatz von 15 mMol Ethylendiamintetraessigsäure gelöst. Nach zweistündigem Inkubieren bie 37 °C trennt man unter Argonschutz über eine Sephadex G 25-Säule ab. Eine Bestimmung der Sulfhydrylgruppen ergibt 238 nMol SH-Gruppen im Reaktionsansatz.

8,3 mg (3,0 µMol) des in Beispiel 11 beschriebenen Komplexbildners werden in 10 ml 0,1 M-Phosphatpuffer (pH 6,0) gelöst. Hierzu fügt man bei 4 °C die oben hergestellte Lösung des Fab-Fragments und läßt über Nacht unter leichtem Schütteln (bei maximal 4 °C) reagieren. Danach eluiert man über einen Kationenaustauscher, dialysiert gegen 0,1 m-Ammoniumacetatlösung und lyophilisiert. Man erhält 13,75 mg eines weißen Pulvers.

1 ml $^{111}InCl_3$-Lösung (pH 5,5, 83 mCi/ml) werden zu einer Lösung des Konjugats in 25 ml Puffer (20 mMol Natriumacetat, 150 mMol Natriumchlorid) gegeben und 4 Stunden inkubiert. Danach fügt man nochmals 5 ml 0,1 m-Natriumacetatlösung zu, dialysiert und lyphylisiert. Man erhält 13,25 mg weißes Pulver mit einer spezifischen Aktivität von 5 mCi/mg.

**BEISPIEL 14**

$^{111}$Indium-Komplex vom Konjugat des monoklonalen Antikörpers 7B10D11 mit 1-(4Hydrazinocarbonylmethoxybenzyl) N,N,N,N-tetrakis-<2-hydroxy-4-oxa-6,10-bis-[di-(carboxymethyl)-amino-8-[(carboxymethyl)-aza]-decyl>-1,2-diaminoethan

30 nMol des Antikörpers werden an einem makroporösen, stark sauren Kationenaustauscher gebunden, der zuvor mit einem 0,1 m-Natriumacetatpuffer (pH 5) äquilibriert wurde und sich in einer durch Aluminiumfolie vor Lichteinfall geschützten Säule befindet. Dann spült man mit 0,03 m Natriumperjodat in Acetatpuffer, bis das Perjodat im Eluat auftaucht. Man unterbricht das Spülen für 30 Minuten, wäscht dann mit Acetatpuffer und zieht dann eine Lösung, die 0,03 m an dem obigen Hydrazid (Beispiel 12) und 0,1 m an Natriumcyanoborhydrid ist auf. Nach 2 Stunden eluiert man nicht gekoppelten Komplexbildner mit Acetatpuffer; das Konjugat wird mit einem Kochsalzgradienten eluiert. Nach Entsalzen wird gefriergetrocknet. Man erhält 4,8 mg Konjugat, das wie in Beispiel 13 beschrieben, in den $^{111}$Indium-Komplex überführt wird.

**Patentansprüche**

1. Verbindungen der allgemeinen Formel I

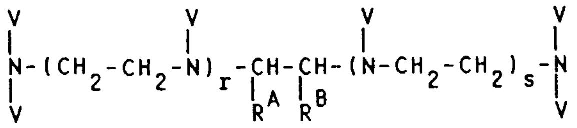

(I)

und

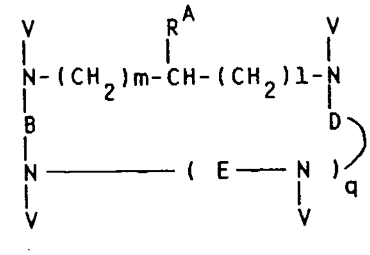

(II),

worin
q für die Ziffern 0, 1 oder 2,
r und s jeweils für die Ziffern 0, 1, 2 und 3,
m für die Ziffern 1, 2, 3, 4 oder 5,
l für die Ziffern 0, 1, 2, 3, 4 oder 5,
$R^A$ und $R^B$ jeweils für ein Wasserstoffatom oder eine gegebenenfalls Imino-, Phenylenoxy-, Phenylenimino-, Amid-, Estergruppe(n) Sauerstoff-, Schwefel-und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe, die am Ende eine funktionelle Gruppe oder gebunden über diese funktionelle Gruppe ein Makromolekül aufweist.
B, D und E, die gleich oder verschieden sind, jeweils für die Gruppe

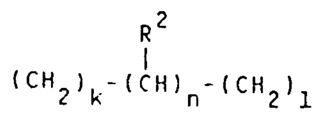

mit
$R^2$ in der Bedeutung von Wasserstoff oder einer gegebenenfalls Sauerstoff-und/oder Stickstoffatom(e) enthaltenden gegebenenfalls durch Hydroxy-und/oder Aminogruppe(n) substituierten geradkettigen, verzweigten, gesättigten oder ungesättigten $C_1$-$C_{20}$-Alkylgruppe
k in der Bedeutung der Ziffern 0, 1, 2, 3, 4 oder 5,
n in der Bedeutung der Ziffern 0 oder 1,
mit der Maßgabe, daß r und s zusammen nicht mehr als 4 ergeben und daß B, D und E jeweils mindestens 2 und maximal 5 Kohlenstoffatom(e) enthält,
V für einen Rest

a) der allgemeinen Formel IA

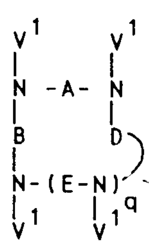

(IA),

worin

A für die Gruppe

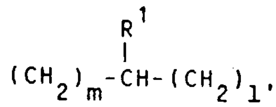

wobei $R^1$ eine gegebenenfalls Imino-, Phenylenoxy-, Phenylenimino-, Amid-, Estergruppe-(n), Sauerstoff-, Schwefel- und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe bedeutet,

$V^1$ für V oder eine $CH_2X$-Gruppe, wobei X die Reste -COOY oder $-PO_3HY$ mit Y in der Bedeutung eines Wasserstoffatoms und/oder eines Metallionenäquivalents eines Elements der Ordnungszahlen 21-29, 31, 32, 38, 39, 42-44 oder 57-83 bedeutet,
oder

b) der allgemeinen Formel IB oder IC

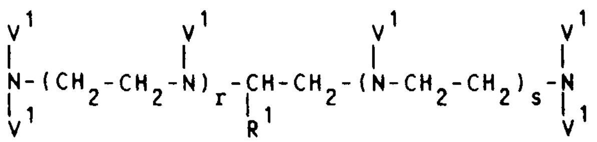

(IB),

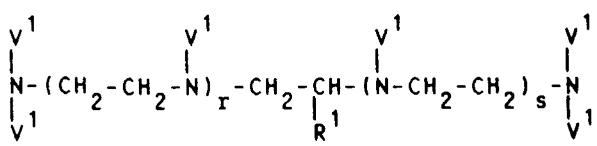

(IC),

wobei die Verknüpfung der Teilstrukturen IA, IB oder IC mit den Stickstoffatomen des Grundgerüstes (I) bzw. (II) über $R^1$ erfolgt,
stehen,
mit der Maßgabe, daß pro Molekül 16 bis 5000 $CH_2X$-Gruppen enthalten sind, sowie deren Salze mit anorganischen und/oder organischen Basen oder Aminosäuren.

**2.** Verbindungen gemäß Anspruch 1, dadurch gekennzeichnet, daß Y Wasserstoffatome darstellt.

**3.** Verbindungen gemäß Anspruch 1, dadurch gekennzeichnet, daß mindestens 2 der Substituenten Y Metallionenäquivalente mindestens eines Elements der Ordnungszahlen 21-29, 42, 44 oder 57-83 sind.

**4.** Verbindungen gemäß Anspruch 1, dadurch gekennzeichnet, daß mindestens zwei der Substituenten Y Metallionenäquivalente eines Radionuklids mindestens eines Elements der Ordnungszahlen 27, 29, 31, 32, 38, 39, 43, 49, 62, 64, 70 oder 77 sind.

**5.** Verbindungen gemäß Anspruch 1, dadurch gekennzeichnet, daß $R^A$ für ein Wasserstoffatom und $R^B$ für eine gegebenenfalls Imino-, Phenylenoxy-, Phenylenimino-, Amid-, Estergruppe(n), Sauerstoff-, Schwefel- und/oder Stickstoff-Atom(e) enthaltende, gegebenenfalls durch Hydroxy-, Mercapto-, Imino- und/ oder Aminogruppe(n) substituierte geradkettige, verzweigte, gesättigte oder ungesättigte $C_1$-$C_{20}$-Alkylengruppe, die am Ende als funktionelle Gruppe $-NH_2$; -NHR; $-NHNH_2$, $NRNH_2$, -SH, -OH, $COCH_3$, $CH=CH-CO_2R$,

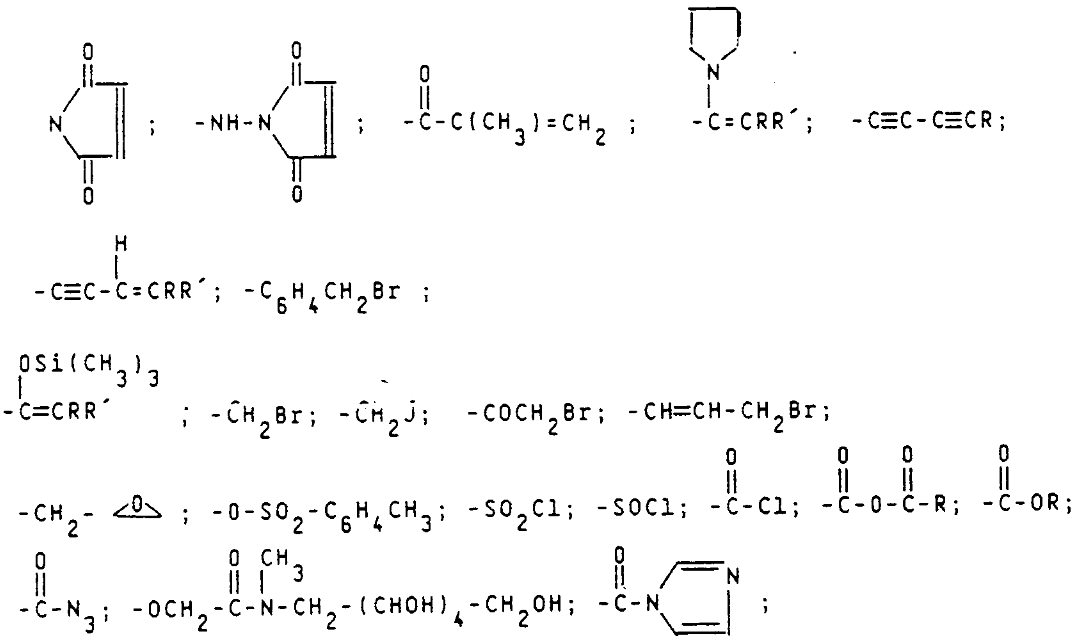

wobei R und R′ gleich oder verschieden sind und jeweils ein Wasserstoffatom, einen gesättigten oder ungesättigten gegebenenfalls durch eine Phenylgruppe substituierten $C_1$-$C_{20}$-Alkylrest oder eine Phenylgruppe bedeuten, aufweist, steht.

6. Verbindungen gemäß Anspruch 1, dadurch gekennzeichnet, daß $R^B$ für ein Wasserstoffatom und $R^A$ für eine gegebenenfalls Imino-, Phenylenoxy-, Phenylenimino-, Amid-, Estergruppe(n), Sauerstoff-, Schwefel und/oder Stickstoff-Atom(e) enthaltende, gegebenenfalls durch Hydroxy-, Mercapto-, Imino-und/oder Aminogruppe(n) substituierte geradkettige, verzweigte, gesättigte oder ungesättigte $C_1$-$C_{20}$-Alkylengruppe, die am Ende als funktionelle Gruppe - $NH_2$; -NHR; -$NHNH_2$, $NRNH_2$,-SH, -OH, $COCH_3$, $CH=CH-CO_2R$,

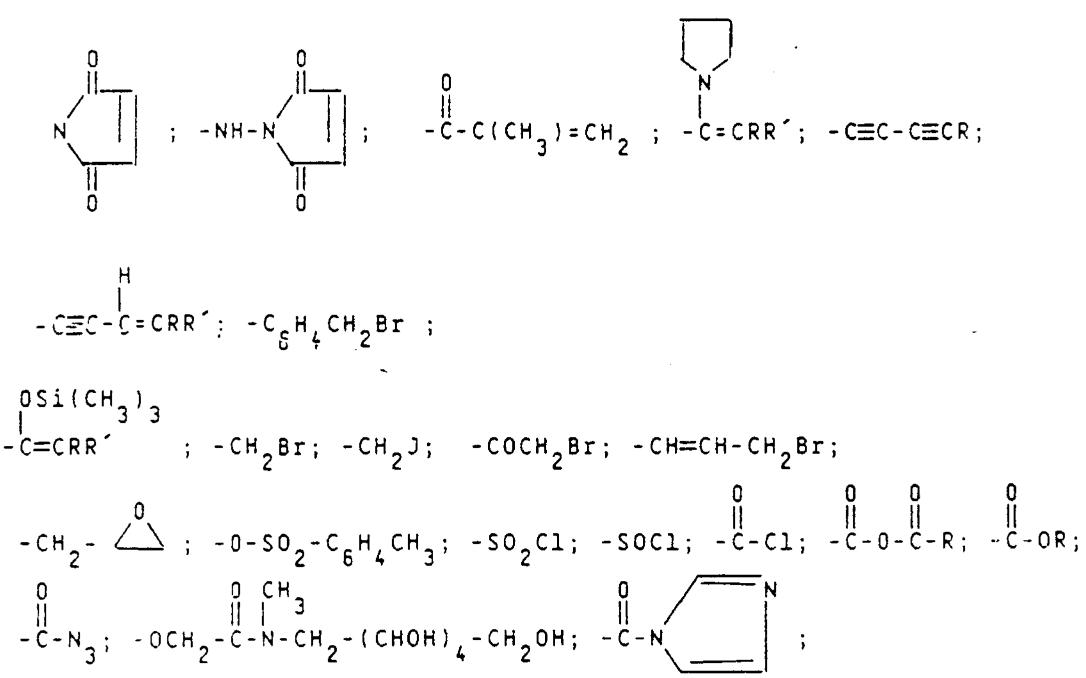

wobei R und R′ gleich oder verschieden sind und jeweils ein Wasserstoffatom, einen gesättigten oder ungesättigten gegebenenfalls durch eine Phenylgruppe substituierten $C_1$-$C_{20}$-Alkylrest oder eine Phenylgruppe bedeuten, aufweist, steht.

**7.** Verbindungen gemäß Anspruch 1, dadurch gekennzeichnet, daß $R^B$ für ein Wasserstoffatom und $R^A$ für eine gegebenenfalls Imino-, Phenylenoxy-, Phenylenimino-, Amid, Estergruppe(n), Sauerstoff-, Schwefel- und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe, die am Ende einen über eine funktionelle Gruppe gebundenen Antikörper oder ein Antikörper-Fragment aufweist, steht.

**8.** Verbindungen gemäß Anspruch 1, dadurch gekennzeichnet, daß $R^A$ für ein Wasserstoffatom und $R^B$ für eine gegebenenfalls Imino-, Phenylenoxy-, Phenylenimino-, Amid-, Estergruppe(n), Sauerstoff-, Schwefel- und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe, die am Ende einen über eine funktionelle Gruppe gebundenen Antikörper oder ein Antikörper-Fragment aufweist, steht.

**9.** Verbindungen der allgemeinen Formel I′ und II′

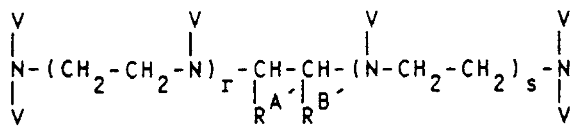

(I′),

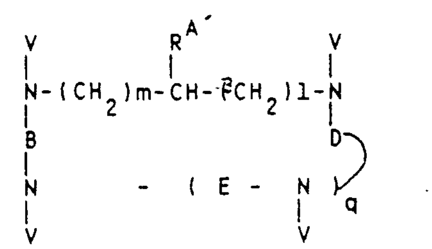

(II′),

worin
r, s, m, l, q, B, D, E und V die in Anspruch 1 genannte Bedeutung haben, $R^{A'}$ und $R^{B'}$ die gleiche Bedeutung wie $R^A$ und $R^B$ haben, mit der Ausnahme, daß kein Makromolekül an die funktionelle Gruppe am Ende der $C_1$-$C_{20}$-Alkylengruppe gebunden sein soll, mit der Maßgabe, daß mindestens zwei der Substituenten X Metallionenäquivalente mindestens eines Elements der in Anspruch 1 genannten Ordnungszahlen sind und daß die Substituenten $R^{A'}$ und $R^{B'}$ verschieden sind und einer für ein Wasserstoffatom und der andere für die oben genannte Alkylengruppe steht,
sowie deren Salze mit anorganischen und/oder organischen Basen oder Aminosäuren.

**10.** Pharmazeutische Mittel, enthaltend mindestens eine physiologisch verträgliche Verbindung nach Anspruch 1 bis 9, gegebenenfalls mit den in der Galenik üblichen Zusätzen.

**11.** Verwendung von mindestens einer physiologisch verträglichen Verbindung gemäß Anspruch 3 für die Herstellung von Mitteln für die NMR-, Röntagen-oder Ultraschall-Diagnostik.

**12.** Verwendung von mindestens einer physiologisch verträglichen Verbindung gemäß Anspruch 4 für die Herstellung von Mitteln für die Radio-Diagnostik.

**13.** Verwendung von mindestens einer physiologisch verträglichen Verbindung gemäß Anspruch 4 für die Herstellung von Mitteln für die Radio-Therapie.

**14.** Verwendung von mindestens einer physiologisch verträglichen Verbindung der allgemeinen Formel I′ oder II′ gemäß Anspruch 9 als Hapten für die Herstellung von Antikörpern.

**15.** Verfahren zur Herstellung von Verbindungen der allgemeinen Formel I

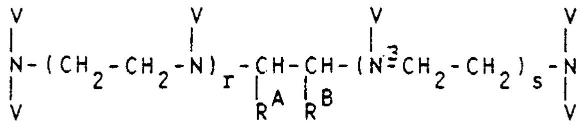

(I)

und

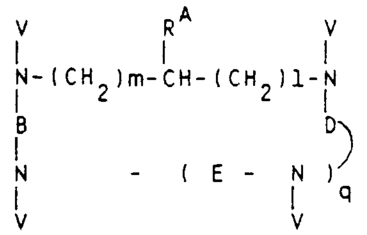

(II),

worin
q für die Ziffern 0, 1 oder 2,
r und s jeweils für die Ziffern 0, 1, 2 und 3,
m für die Ziffern 1, 2, 3, 4, oder 5,
l für die Ziffern 0, 1, 2, 3, 4 oder 5,
$R^A$ und $R^B$ jeweils für ein Wasserstoffatom oder eine gegebenenfalls Imino-, Phenylenoxy-, Phenylenimino-, Amid-, Estergruppe(n), Sauerstoff-, Schwefel-und/oder Stickstoff-Atom(e) enthaltende gegebenenfalls durch Hydroxy-, Mercapto-, Imino- und/oder Aminogruppe(n) substituierte geradkettige, verzweigte, gesättigte oder ungesättigte $C_1$-$C_{20}$-Alkylengruppe, die am Ende eine funktionelle Gruppe oder gebunden über diese funktionelle Gruppe ein Makromolekül aufweist,
B, D und E, die gleich oder verschieden sind, jeweils für die Gruppe

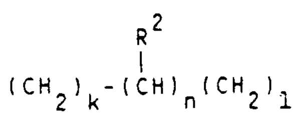

mit
$R^2$ in der Bedeutung von Wasserstoff oder einer gegebenenfalls Sauerstoff-und/oder Stickstoffatom(e) enthaltenden gegebenenfalls durch Hydroxy-und/oder Aminogruppe(n) substituierten geradkettigen, verzweigten, gesättigten oder ungesättigten $C_1$-$C_{20}$-Alkylgruppe
k in der Bedeutung der Ziffern 0, 1, 2, 3, 4 oder 5,
n in der Bedeutung der Ziffern 0 oder 1,
mit der Maßgabe, daß r und s zusammen nicht mehr als 4 ergeben und daß B, D und E jeweils mindestens 2 und maximal 5 Kohlenstoffatom(e) enthält,
V für einen Rest
a) der allgemeinen Formel IA

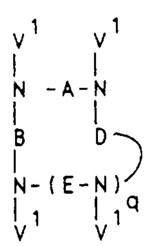

(IA),

worin

A für die Gruppe

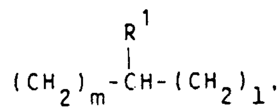

wobei $R^1$ eine gegebenenfalls Imino-, Phenylenoxy-, Phenylenimino-, Amid-, Estergruppe-(n), Sauerstoff-, Schwefel- und/oder Stickstoff-Atom(e) enthaltende gegebenenfalls durch Hydroxy-, Mercapto-,Imino- und/oder Aminogruppe(n) substituierte geradkettige, verzweigte, gesättigte oder ungesättigte $C_1$-$C_{20}$-Alkylengruppe bedeutet,

$V^1$ für V oder eine $CH_2X$-Gruppe,
wobei X die Reste -COOY oder -$PO_3HY$ mit Y in der Bedeutung eines Wasserstoffatoms und/oder eines Metallionenäquivalents eines Elements der Ordnungszahlen 21-29, 31, 32, 38, 39, 42-44 oder 57-83 bedeutet,
oder

b) der allgemeinen Formel IB oder IC

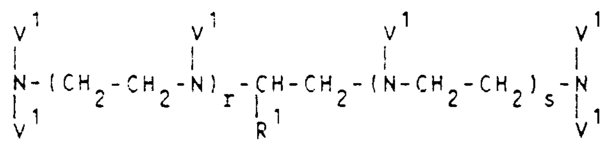 (IB),

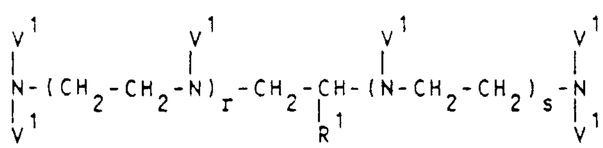 (IC),

stehen,
mit der Maßgabe, daß pro Molekül 16 bis 5000 $CH_2X$-Gruppen enthalten sind,
sowie deren Salze mit anorganischen und/oder organischen Basen oder Amino säuren,
dadurch gekennzeichnet, daß man in an sich bekannter Weise eine Verbindung der allgemeinen Formel III oder IV

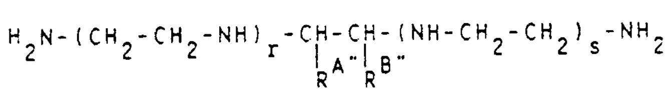 (III),

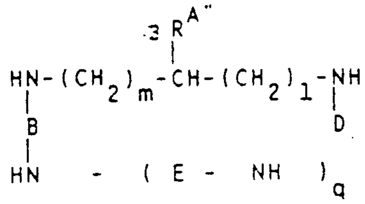 (IV),

worin r, s, m, l, q, B, D und E die oben genannte Bedeutung haben und $R^{A''}$ und $R^{B''}$ jeweils für ein Wasserstoffatom oder einen Substituenten, der in $R^{A'}$ bzw. $R^{B'}$ umgewandelt werden kann,
stehen, mit der Maßgabe, daß r und s zusammen nicht mehr als 4 ergeben, mit einem den Rest V einführenden Reaktionspartner umsetzt,
die so, gegebenenfalls nach Abspaltung von Säureschutzgruppen $Y'$, erhaltenen Säuren der allgemeinen Formel I mit Y in der Bedeutung eines Wasserstoffatoms gewünschtenfalls

a) in an sich bekannter Weise mit mindestens einem Metalloxid oder Metallsalz eines Elements der Ordnungszahlen 21-29, 31, 32, 38, 39,42-44, 49 oder 57-83 umsetzt und anschließend, falls gewünscht, vorhandene azide Wasserstoffatome durch Kationen anorganischer und/oder organischer Basen oder Aminosäuren substituiert,

oder

b) in an sich bekannter Weise mit mindestens einem Metalloxid oder Metallsalz eines Elements der Ordnungszahlen 21-29, 31, 32, 38, 39, 42-44, 49, oder 57-83 umsetzt und anschließend die so erhaltenen Metallkomplexe in an sich bekannter Weise über die in $R^{A'}$ bzw. $R^{B'}$ enthaltene funktionelle Gruppe an ein Makromolekül bindet und, falls gewünscht, vorhandene azide Wasserstoffatome durch Kationen anorganischer und/oder organischer Basen oder Aminosäuren substituiert,

oder

c) in an sich bekannter Weise über die in $R^{A'}$ bzw. $R^{B'}$ enthaltene funktionelle Gruppe an ein Makromolekül bindet und anschließend in an sich bekannter Weise mit mindestens einem Metalloxid oder Metallsalz eines Elements der Ordnungszahlen 21-29, 31, 32, 38, 39, 42-44, 49 oder 57-83 umsetzt und anschließend, falls gewünscht, vorhandene azide Wasserstoffatome durch Kationen anorganischer und/oder organischer Basen oder Aminosäuren substituiert.

**16.** Verfahren gemäß Anspruch 15, dadurch gekennzeichnet, daß man die Kopplung des Makromoleküls an den funktionalisierten Komplex oder Liganden sowie, im Falle der Kopplung an den Liganden, die nachfolgende Komplexierung mit dem/den gewünschten Metallion(en) an einer stationären Phase durchführt.

**17.** Verfahren zur Herstellung der pharmazeutischen Mittel gemäß Anspruch 10,dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

**1.** Compounds of the general formula I

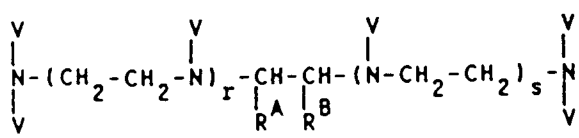

(I)

and

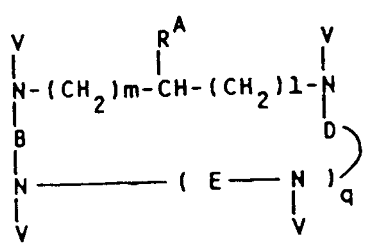

(II)

in which

q represents 0, 1 or 2,

each of r and s represents 0, 1, 2 or 3,

m represents 1, 2, 3, 4 or 5,

l represents 0, 1, 2, 3, 4 or 5,

each of $R^A$ and $R^B$ represents a hydrogen atom or a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group(s), and that has on its end a functional group or a

macromolecule bonded <u>via</u> that functional group,
each of B, D and E, which are the same or different, represents the group

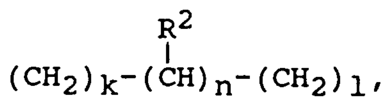

wherein
$R^2$ represents hydrogen or a straight-chained, branched, saturated or unsaturated $C_1$-$C_{20}$-alkyl group that optionally contains oxygen and/or nitrogen atom(s) and is optionally substituted by hydroxy and/or amino group(s),
k represents 0, 1, 2, 3, 4 or 5,
n represents 0 or 1,
with the proviso that r and s together do not add up to more than 4 and that each of B, D and E contains at least 2 and a maximum of 5 carbon atoms,
V represents a radical
a) of the general formula IA

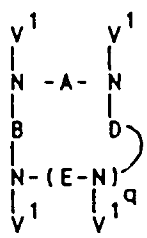

(IA)

in which
A represents the group

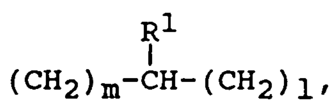

wherein $R^1$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group(s),
$V^1$ represents V or a $CH_2X$ group,
wherein X represents a radical -COOY or $-PO_3HY$, wherein Y represents a hydrogen atom and/or a metal ion equivalent of an element of atomic numbers 21-29, 31, 32, 38, 39, 42-44 or 57-83, or
b) of the general formula IB or IC

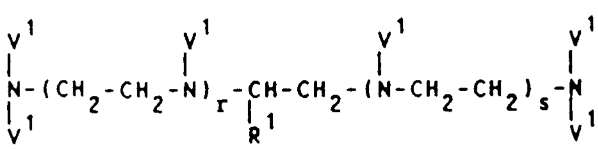

(IB)

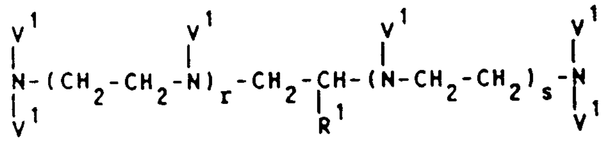 (IC),

the partial structures IA, IB or IC being linked to the nitrogen atoms of the basic structure (I) or (II) via $R^1$, with the proviso that from 16 to 5000 $CH_2X$ groups are present per molecule, and salts thereof with inorganic and/or organic bases or amino acids.

2. Compounds according to claim 1, characterised in that Y's represent hydrogen atoms.

3. Compounds according to claim 1, characterised in that at least two of the Y substituents are metal ion equivalents of at least one element of atomic numbers 21-29, 42, 44 or 57-83.

4. Compounds according to claim 1, characterised in that at least two of the Y substituents are metal ion equivalents of a radionuclide of at least one element of atomic numbers 27, 29, 31, 32, 38, 39, 43, 49, 62, 64, 70 or 77.

5. Compounds according to claim 1, characterised in that $R^A$ represents a hydrogen atom and $R^B$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group-(s), and that has on its end as functional group $-NH_2$; $-NHR$; $-NHNH_2$; $NRNH_2$; $-SH$; $-OH$; $COCH_3$; $CH=CH-CO_2R$;

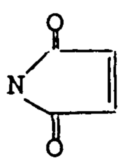 ; 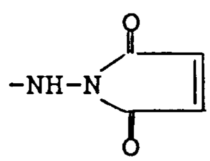 ; 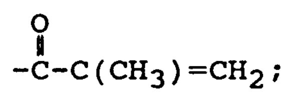 ; 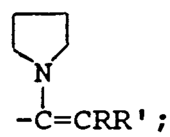 ;

$-C \equiv C-C \equiv CR$; 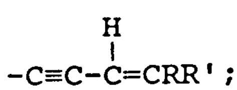 ; $-C_6H_4CH_2Br$; 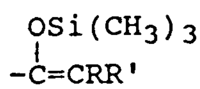 ;

$-CH_2Br$; $-CH_2I$; $-COCH_2Br$; $-CH=CH-CH_2Br$; 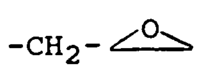 ;

$-O-SO_2-C_6H_4CH_3$; $-SO_2Cl$; $-SOCl$; 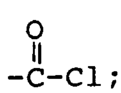 ; 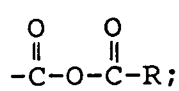 ;

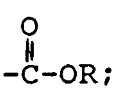 ; 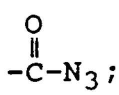 ; 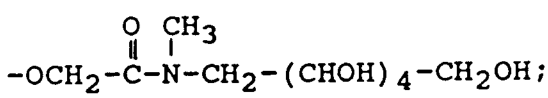 ; 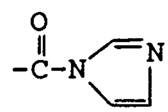 ;

in which R and R' are the same or different and each represents a hydrogen atom, a saturated or unsaturated $C_1$-$C_{20}$-alkyl radical optionally substituted by a phenyl group, or a phenyl group.

6. Compounds according to claim 1, characterised in that $R^B$ represents a hydrogen atom and $R^A$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group-(s), and that has on its end as functional group $-NH_2$; $-NHR$; $-NHNH_2$; $NRNH_2$; $-SH$; $-OH$; $COCH_3$; $CH=CH-CO_2R$;

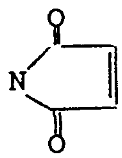 ; 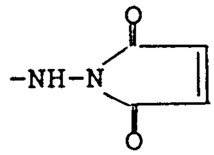 ; 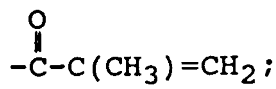 ; 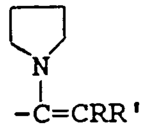 ;

$-C\equiv C-C\equiv CR$; 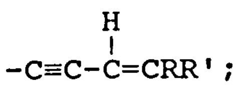 ; $-C_6H_4CH_2Br$; 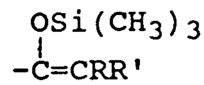 ;

$-CH_2Br$; $-CH_2I$; $-COCH_2Br$; $-CH=CH-CH_2Br$; 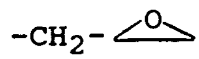 ;

$-O-SO_2-C_6H_4CH_3$; $-SO_2Cl$; $-SOCl$; 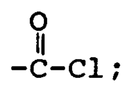 ; 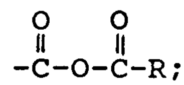 ;

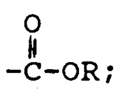 ; 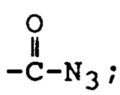 ; 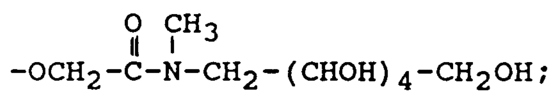 ; 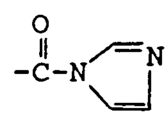 ;

in which R and R' are the same or different and each represents a hydrogen atom, a saturated or unsaturated $C_1$-$C_{20}$-alkyl radical optionally substituted by a phenyl group, or a phenyl group.

7. Compounds according to claim 1, characterised in that $R^B$ represents a hydrogen atom and $R^A$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group-(s), and that has on its end an antibody or antibody fragment bonded via a functional group.

8. Compounds according to claim 1, characterised in that $R^A$ represents a hydrogen atom and $R^B$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group-(s), and that has on its end an antibody or antibody fragment bonded via a functional group.

9. Compounds of the general formulae I' and II'

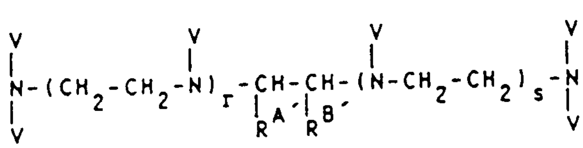 (I')

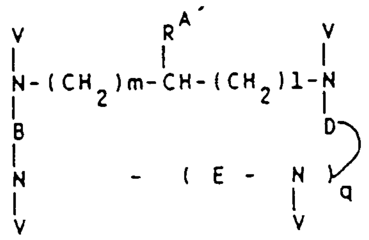

(II')

in which
r, s, m, l, q, B, D, E and V are as defined in claim 1, and $R^{A'}$ and $R^{B'}$ have the same meanings as $R^A$ and $R^B$,except that no macromolecule is to be bonded to the functional group on the end of the $C_1$-$C_{20}$-alkylene group, with the proviso that at least two of the X substituents are metal ion equivalents of at least one element of the atomic numbers mentioned in claim 1, and that the substituents $R^{A'}$ and $R^{B'}$ are different and one represents a hydrogen atom and the other represents the alkylene group mentioned above, and salts thereof with inorganic and/or organic bases or amino acids.

**10.** Pharmaceutical agent comprising at least one physiologically compatible compound according to any one of claims 1 to 9, optionally together with the additives customary in galenics.

**11.** Use of at least one physiologically compatible compound according to claim 3 for the manufacture of agents for NMR, X-ray or ultrasound diagnostics.

**12.** Use of at least one physiologically compatible compound according to claim 4 for the manufacture of agents for radiodiagnostics.

**13.** Use of at least one physiologically compatible compound according to claim 4 for the manufacture of agents for radiotherapy.

**14.** Use of at least one physiologically compatible compound of the general formula I' or II' according to claim 9 as a hapten for the production of antibodies.

**15.** Process for the manufacture of compounds of the general formula I

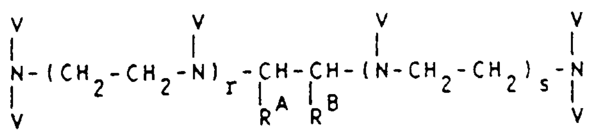

(I)

and

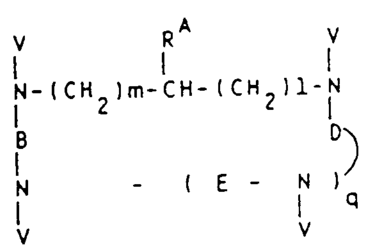

(II),

in which
q represents 0, 1 or 2,
each of r and s represents 0, 1, 2 or 3,
m represents 1, 2, 3, 4 or 5,
l represents 0, 1, 2, 3, 4 or 5,
each of $R^A$ and $R^B$ represents a hydrogen atom or a straight-chained, branched, saturated or unsaturated $C_1$-$C_{20}$-alkylene group that optionally contains imino, phenyleneoxy, phenyleneimino,

amide and/or ester group(s) and/or oxygen, sulphur and/or nitrogen atom(s) and is optionally substituted by hydroxy, mercapto, imino and/or amino group(s), and that has on its end a functional group or a macromolecule bonded via that functional group,
each of B, D and E, which are the same or different, represents the group

$$(CH_2)_k-(\overset{\overset{R^2}{|}}{C}H)_n(CH_2)_l,$$

wherein $R^2$ represents hydrogen or a straight-chained, branched, saturated or unsaturated $C_1$-$C_{20}$-alkyl group that optionally contains oxygen and/or nitrogen atom(s) and is optionally substituted by hydroxy and/or amino group(s),
k represents 0, 1, 2, 3, 4 or 5,
n represents 0 or 1,
with the proviso that r and s together do not add up to more than 4 and that each of B, D and E contains at least 2 and a maximum of 5 carbon atoms,
V represents a radical
a) of the general formula IA

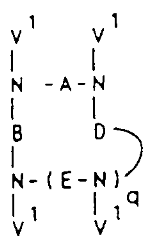

(IA)

in which
A represents the group

$$(CH_2)_m-\overset{\overset{R^1}{|}}{C}H-(CH_2)_l,$$

wherein $R^1$ represents a straight-chained, branched, saturated or unsaturated $C_1$-$C_{20}$-alkylene group that optionally contains imino, phenyleneoxy, phenyleneimino, amide and/or ester group(s) and/or oxygen, sulphur and/or nitrogen atom(s) and is optionally substituted by hydroxy, mercapto, imino and/or amino group(s),
$V^1$ represents V or a $CH_2X$ group,
wherein X represents a radical -COOY or $-PO_3HY$, wherein Y represents a hydrogen atom and/or a metal ion equivalent of an element of atomic numbers 21-29, 31, 32, 38, 39, 42-44 or 57-83, or
b) of the general formula IB or IC

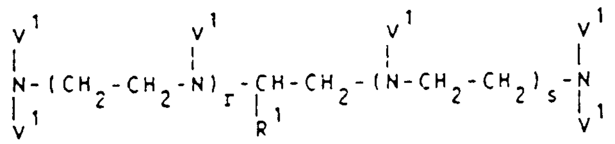

(IB)

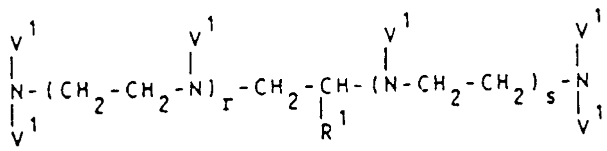

(IC),

with the proviso that from 16 to 5000 $CH_2X$ groups are present per molecule, and salts thereof with inorganic and/or organic bases or amino acids,
characterised in that a compound of the general formula III or IV

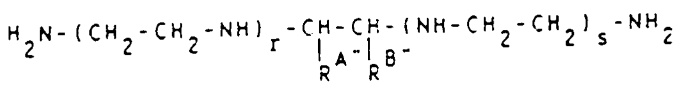

(III)

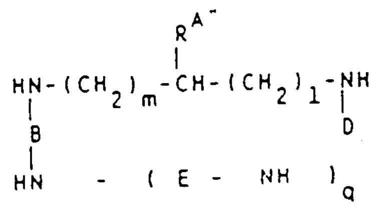

(IV),

in which r, s, m, l, q, B, D and E are as defined above and each of $R^{A''}$ and $R^{B''}$ represents a hydrogen atom or a substituent that can be converted into $R^{A'}$ or $R^{B'}$, respectively,
with the proviso that r and s together do not add up to more than 4,
is reacted in a manner known per se with a reactant introducing the radical V, and, if required after removal of acid-protecting groups Y', the resulting acids of the general formula I in which Y represents a hydrogen atom are, if desired,

a) reacted in a manner known per se with at least one metal oxide or metal salt of an element of atomic numbers 21-29, 31, 32, 38, 39, 42-44, 49 or 57-83, and then, if desired, any acidic hydrogen atoms present are replaced by cations of inorganic and/or organic bases or amino acids,
or
b) reacted in a manner known per se with at least one metal oxide or metal salt of an element of atomic numbers 21-29, 31, 32, 38, 39, 42-44, 49 or 57-83, and then the metal complexes so obtained are bonded in a manner known per se to a macromolecule via the functional group contained in $R^{A'}$ or $R^{B'}$, and, if desired, any acidic hydrogen atoms present are replaced by cations of inorganic and/or organic bases or amino acids,
or
c) bonded in a manner known per se to a macromolecule via the functional group contained in $R^{A'}$ or $R^{B'}$ and then reacted in a manner known per se with at least one metal oxide or metal salt of an element of atomic numbers 21-29, 31, 32, 38, 39, 42-44, 49 or 57-83, and then, if desired, any acidic hydrogen atoms present are replaced by cations of inorganic and/or organic bases or amino acids.

**16.** Process according to claim 15, characterised in that the coupling of the macromolecule to the functionalised complex or ligand and; in the case of coupling to the ligand, the subsequent complexing with the desired metal ion(s) are carried out on a stationary phase.

**17.** Process for the manufacture of the pharmaceutical agents according to claim 10, characterised in that

the complex compound dissolved or suspended in water or physiological saline solution, optionally together with the additives customary in galenics, is converted into a form suitable for enteral or parenteral administration.

## Revendications

**1.** Composés de formules générales I et II ci-dessous :

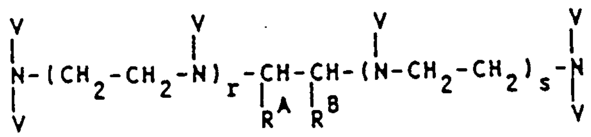

(I)

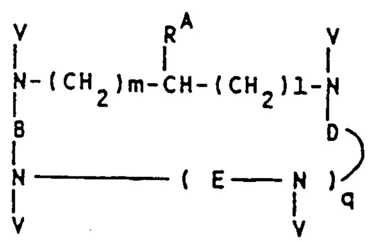

(II)

dans lesquelles

| | |
|---|---|
| q | désigne le nombre 0, 1 ou 2, |
| r et s | désignent chacun le nombre 0, 1, 2 ou 3, |
| m | désigne le nombre 1, 2, 3, 4 ou 5, |
| l | désigne le nombre 0, 1, 2, 3, 4 ou 5, |
| $R^A$ et $R^B$ | représentent chacun un atome d'hydrogène ou un groupe alkylène en $C_1$-$C_{20}$ linéaire ou ramifié, saturé ou insaturé, ayant éventuellement un ou plusieurs groupes imino, phénylène-oxy, phénylène-imino, amide ou ester et un ou plusieurs atomes d'oxygène, de soufre et/ou d'azote, et le cas échéant comme substituants un ou plusieurs groupes hydroxy, mercapto, imino et/ou amino, alkylène qui porte à son extrémité un groupe fonctionnel ou une macromolécule liée par ce groupe fonctionnel, |
| B, D et E, | qui peuvent être identiques ou différents les uns des autres, représentent chacun un groupe |

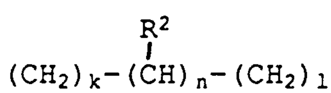

$R^2$ étant l'hydrogène ou un alkyle en $C_1$-$C_{20}$ linéaire ou ramifié, saturé ou insaturé, avec éventuellement un ou plusieurs atomes d'oxygène et/ou d'azote et le cas échéant comme substituants un ou plusieurs groupes hydroxy et/ou amino,
k étant le nombre 0, 1, 2, 3, 4 ou 5, et
n le nombre 0 ou 1,

avec les conditions que la somme r + s ne dépasse pas 4 et que B, D et E aient chacun au moins 2 et au maximum 5 atomes de carbone,

V désigne un radical

a) de formule générale IA

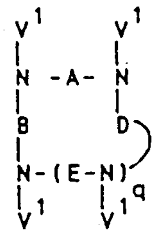

(IA)

dans laquelle

A est un groupe

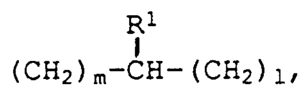

$R^1$ désignant un alkylène en $C_1$-$C_{20}$ linéaire ou ramifié, saturé ou insaturé, avec éventuellement un ou plusieurs groupes imino, phénylène-oxy, phénylène-imino, amide ou ester et/ou plusieurs atomes d'oxygène, de soufre et/ou d'azote, et le cas échéant comme substituants un ou plusieurs groupes hydroxy, mercapto, imino et/ou amino, et

$V^1$ représentant V ou un groupe $CH_2X$,

X étant le radical -COO ou $-PO_3HY$, Y représentant un atome d'hydrogène et/ou un équivalent d'ions de métaux d'un élément de numéro atomique 21-29, 31, 32, 38, 39, 42-44 ou 57-83,

ou bien

b) de formule générale IB ou IC

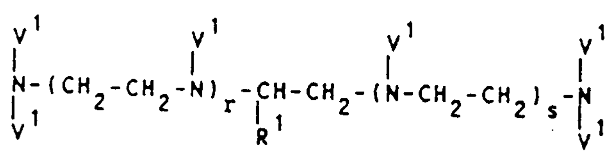

(IB)

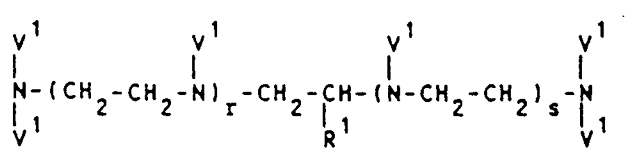

(IC)

la liaison des radicaux IA, IB et IC avec les atomes d'azote des formules I et II se faisant par $R^1$, avec la condition qu'il y ait par molécule de 16 à 5000 groupes $CH_2X$-, ainsi que les sels de ces composés formés avec des bases minérales et/ou organiques ou avec des acides aminés.

**2.** Composés selon la revendication 1, caractérisés en ce que Y représente des atomes d'hydrogène.

**3.** Composés selon la revendication 1, caractérisés en ce qu'au moins deux des substituants Y sont des équivalents d'ions de métaux d'un ou de plusieurs éléments des numéros atomiques 21-29, 42, 44 ou 57-83.

**4.** Composés selon la revendication 1, caractérisés en ce qu'au moins deux des substituants Y sont des

équivalents d'ions de métaux d'un radionucléide d'un ou de plusieurs éléments des numéros atomiques 27, 29, 31, 32, 38, 39, 43, 49, 62, 64, 70 ou 77.

**5.** Composés selon la revendication 1, caractérisés en ce que $R^A$ est un atome d'hydrogène et $R^B$ un groupe alkylène en $C_1$-$C_{20}$ linéaire ou ramifié, saturé ou insaturé, avec éventuellement un ou plusieurs groupes imino, phénylène-oxy, phénylène-imino, amide ou ester et un ou plusieurs atomes d'oxygène, de soufre et/ou d'azote, groupe alkylène qui peut aussi le cas échéant comporter comme substituants un ou plusieurs groupes hydroxy, mercapto, imino et/ou amino et qui porte à son extrémité l'un des groupes suivants comme groupe fonctionnel : $-NH_2$ ; -NHR ; $-NHHH_2$, $NRNH_2$, -SH, -OH, $COCH_3$, $CH=CH-CO_2R$,

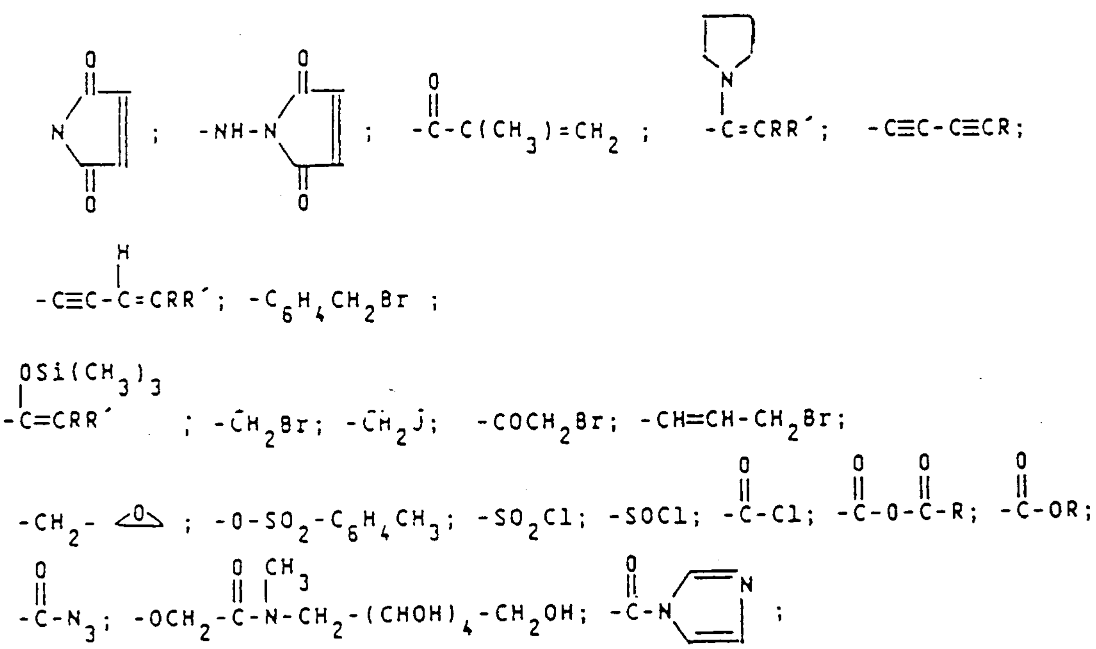

R et R', qui peuvent être identiques ou différents l'un de l'autre, désignant chacun un atome d'hydrogène, un alkyle en $C_1$-$C_{20}$ saturé ou insaturé, éventuellement substitué par un groupe phényle, ou bien le groupe phényle.

**6.** Composés selon la revendication 1, caractérisés en ce que $R^B$ est un atome d'hydrogène et $R^A$ un groupe alkylène en $C_1$-$C_{20}$ linéaire ou ramifié, saturé ou insaturé, avec éventuellement un ou plusieurs groupes imino, phénylène-oxy, phénylène-imino, amide ou ester et un ou plusieurs atomes d'oxygène, de soufre et/ou d'azote, groupe alkylène qui peut aussi le cas échéant comporter comme substituants un ou plusieurs groupes hydroxy, mercapto, imino et/ou amino et qui porte à son extrémité l'un des groupes suivants comme groupe fonctionnel : $-NH_2$ ; -NHR ; $-NHHH_2$, $NRNH_2$, -SH, -OH, $COCH_3$, $CH=CH-CO_2R$,

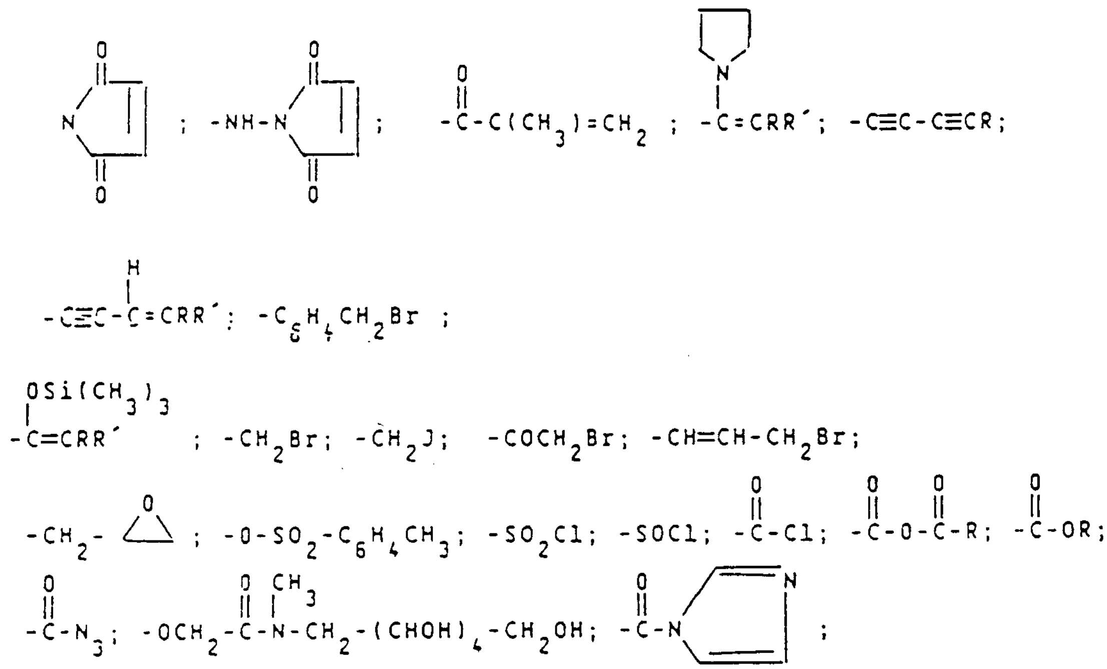

R et R', qui peuvent être identiques ou différents l'un de l'autre, désignant chacun un atome d'hydrogène, un alkyle en $C_1$-$C_{20}$ saturé ou insaturé, éventuellement substitué par un groupe phényle, ou bien le groupe phényle.

**7.** Composés selon la revendication 1, caractérisés en ce que $R^B$ est un atome d'hydrogène et $R^A$ un groupe alkylène en $C_1$-$C_{20}$ linéaire ou ramifié, saturé ou insaturé, avec éventuellement un ou plusieurs groupes imino, phénylène-oxy, phénylène-imino, amide ou ester et un ou plusieurs atomes d'oxygène, de soufre et/ou d'azote, groupe alkylène qui peut aussi le cas échéant comporter comme substituants un ou plusieurs groupes hydroxy, mercapto, imino et/ou amino et qui porte à son extrémité un anticorps ou un fragment d'anticorps lié par l'intermédiaire d'un groupe fonctionnel.

**8.** Composés selon la revendication 1, caractérisés en ce que $R^A$ est un atome d'hydrogène et $R^B$ un groupe alkylène en $C_1$-$C_{20}$ linéaire ou ramifié, saturé ou insaturé, avec éventuellement un ou plusieurs groupes imino, phénylène-oxy, phénylène-imino, amide ou ester et un ou plusieurs atomes d'oxygène, de soufre et/ou d'azote, groupe alkylène qui peut aussi le cas échéant comporter comme substituants un ou plusieurs groupes hydroxy, mercapto, imino et/ou amino et qui porte à son extrémité un anticorps ou un fragment d'anticorps lié par l'intermédiaire d'un groupe fonctionnel.

**9.** Composés de formules générales I' et II' ci-dessous :

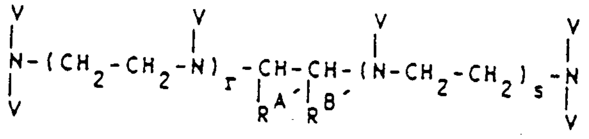

(I'),

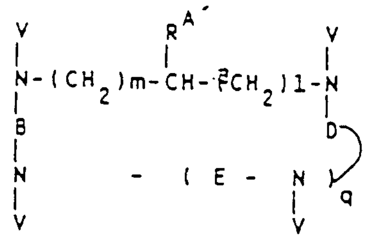

(II'),

formules dans lesquelles
r, s, m, l, q, B, D, E et V ont les significations précédemment indiquées à la revendication 1 et $R^{A'}$ et $R^{B'}$ les mêmes significations que $R^A$ et $R^B$, sauf qu'il n'y a pas de macromolécule liée au groupe fonctionnel à l'extrémité de l'alkylène en $C_1$-$C_{20}$, avec la condition qu'au moins deux des substituants X soient des équivalents d'ions de métaux d'un ou de plusieurs des éléments des numéros atomiques qui ont été indiqués à la revendication 1, et aussi que les substituants $R^{A'}$ et $R^{B'}$ soient différents, l'un des deux étant un atome d'hydrogène et l'autre le groupe alkylène ci-dessus indiqué, ainsi que les sels de ces composés formés avec des bases minérales et/ou organiques ou avec des acides aminés.

**10.** Produits pharmaceutiques comprenant un ou plusieurs composés physiologiquement tolérés selon les revendications 1 à 9, avec éventuellement les additifs courants en pharmacie galénique.

**11.** L'emploi des composés de la revendication 3 physiologiquement tolérés pour préparer des agents de diagnostic par RMN, radiographies X ou ultrasons.

**12.** L'emploi des composés de la revendication 4 physiologiquement tolérés pour préparer des agents de radiodiagnostic (radioactifs).

**13.** L'emploi des composés de la revendication 4 physiologiquement tolérés pour préparer des produits de traitement radiothérapeutique (radioactifs).

**14.** L'emploi des composés physiologiquement tolérés des formules I' et II' de la revendication 9 comme haptènes pour la préparation d'anticorps.

**15.** Procédé de préparation des composés de formules générales I et II ci-dessous :

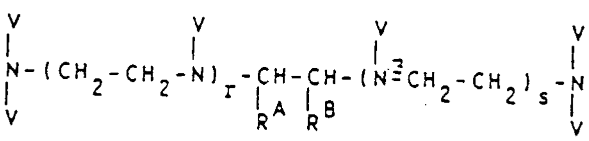

(I)

et

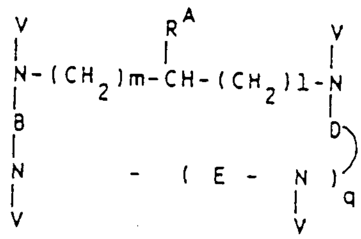

(II)

dans lesquelles

- q désigne le nombre 0, 1 ou 2,
- r et s désignent chacun le nombre 0, 1, 2 ou 3,
- m désigne le nombre 1, 2, 3, 4 ou 5,
- l désigne le nombre 0, 1, 2, 3, 4 ou 5,
- $R^A$ et $R^B$ représentent chacun un atome d'hydrogène ou un groupe alkylène en $C_1$-$C_{20}$ linéaire ou ramifié, saturé ou insaturé, ayant éventuellement un ou plusieurs groupes imino, phénylène-oxy, phénylène-imino, amide ou ester et un ou plusieurs atomes d'oxygène, de soufre et/ou d'azote, et le cas échéant comme substituants un ou plusieurs groupes hydroxy, mercapto, imino et/ou amino, alkylène qui porte à son extrémité un groupe fonctionnel ou une macromolécule liée par ce groupe fonctionnel,
- B, D et E, qui peuvent être identiques ou différents les uns des autres, représentent chacun le groupe

$$(CH_2)_k-\overset{R^2}{\overset{|}{(CH)}}_n-(CH_2)_l$$

$R^2$ étant l'hydrogène ou un alkyle en $C_1$-$C_{20}$ linéaire ou ramifié, saturé ou insaturé, avec éventuellement un ou plusieurs atomes d'oxygène et/ou d'azote et le cas échéant comme substituants un ou plusieurs groupes hydroxy et/ou amino,
k étant le nombre 0, 1, 2, 3, 4 ou 5, et
n le nombre 0 ou 1,

avec les conditions que la somme r + s ne dépasse pas 4 et que B, D et E aient chacun au moins 2 et au maximum 5 atomes de carbone,

V désigne un radical

a) de formule générale IA

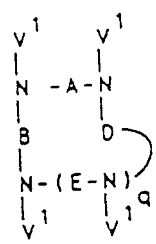

(IA)

dans laquelle

A est un groupe

$$(CH_2)_m-\overset{R^1}{\overset{|}{C}}H-(CH_2)_l,$$

$R^1$ désignant un alkylène en $C_1$-$C_{20}$ linéaire ou ramifié, saturé ou insaturé, avec éventuellement un ou plusieurs groupes imino, phénylène-oxy, phénylène-imino, amide ou ester et/ou plusieurs atomes d'oxygène, de soufre et/ou d'azote, et le cas échéant comme substituants un ou plusieurs groupes hydroxy, mercapto, imino et/ou amino, et

$V^1$ représentant V ou un groupe $CH_2X$,

X étant le radical -COO ou $-PO_3HY$, Y représentant un atome d'hydrogène et/ou un équivalent d'ions de métaux d'un élément de numéro atomique 21-29, 31, 32, 38, 39, 42-44 ou 57-83,

ou bien

b) de formule générale IB ou IC

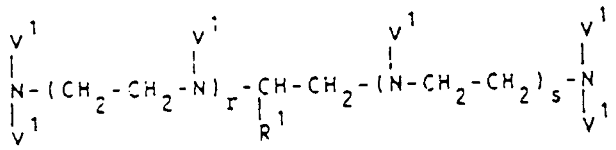

(IB)

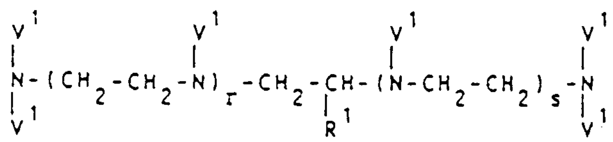

(IC)

avec la condition qu'il y ait par molécule de 16 à 5000 groupes $CH_2X$-,

ainsi que de sels de ces composés formés avec des bases minérales et/ou organiques ou avec des acides aminés,

procédé caractérisé en ce que l'on fait réagir de manière connue un composé de formule générale III ou IV ci-dessous :

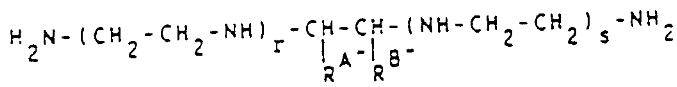

(III)

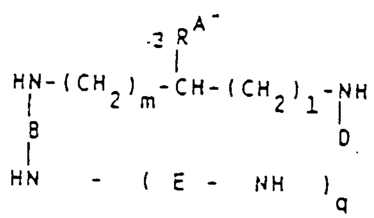

(IV),

dans lesquelles r, s, m, l, q, B, D et E ont les significations précédemment indiquées et $R^{A''}$ et $R^{B''}$ désignent chacun un atome d'hydrogène ou un substituant pouvant être transformé en un groupe $R^{A'}$ et $R^{B'}$,

avec la condition que la somme r + s ne dépasse pas 4,

avec un réactif apportant le radical V,

puis, si on le souhaite, éventuellement après avoir éliminé des groupes protecteurs d'acides Y' :

a) on fait réagir les acides de formule I ainsi formés (Y étant un atome d'hydrogène), de manière connue, avec un ou plusieurs oxydes ou sels de métaux, d'éléments des numéros atomiques 21-29, 31, 32, 38, 39, 42-44, 49 ou 57-83, puis, le cas échéant, on remplace des atomes d'hydrogène d'azides présents par des cations de bases minérales et/ou organiques ou d'aminoacides,

ou bien

b) on fait réagir ces acides de manière connue avec un ou plusieurs oxydes ou sels de métaux, d'éléments des numéros atomiques 21-29, 31, 32, 38, 39, 42-44, 49 ou 57-83, puis, de manière connue on lie à une macromolécule les complexes de métaux ainsi formés par le groupe fonctionnel

de $R^{A'}$ et/ou $R^{B'}$, et le cas échéant on remplace des atomes d'hydrogène d'azides présents par des cations de bases minérales et/ou organiques ou d'acides aminés,
ou encore
c) on lie ces acides de manière connue à une macromolécule par le groupe fonctionnel de $R^{A'}$ et/ou $R^{B'}$ puis, en procédant toujours de manière connue, on les fait réagir avec un ou plusieurs oxydes ou sels de métaux d'éléments des numéros atomiques 21-29, 31, 32, 38, 39, 42-44, 49 ou 57-83, et le cas échéant on remplace ensuite de atomes d'hydrogène d'azides présents par des cations de bases minérales et/ou organiques ou d'acides aminés.

**16.** Procédé selon la revendication 15, caractérisé en ce que le couplage de la macromolécule au complexe fonctionnalisé ou à des ligands (coordinats), ainsi que, dans le cas d'un couplage à des ligands, le complexage qui suit avec le ou les ions de métaux, se font sur une phase stationnaire.

**17.** Procédé de préparation des produits pharmaceutiques de la revendication 10, procédé caractérisé en ce que l'on met sous une forme appropriée à une administration entérale ou parentérale le composé complexe en solution ou en suspension dans de l'eau ou dans du sérum physiologique, éventuellement avec les additifs courants en pharmacie galénique.